# EUROPEAN PATENT SPECIFICATION

(11) **EP 0 457 834 B1**
(45) Date of publication and mention of the grant of the patent: **08.06.1994**
(21) Application number: 90903536.2
(22) Date of filing: 08.02.1990
(51) Int. Cl.: A61K 9/10

(54) **SUSTAINED RELEASE DOSAGE FORM**
DOSIERUNGSFORM FÜR DAUERVERABREICHUNG
FORME DE DOSAGE A LIBERATION ETALEE

(30) Priority: 08.02.1989 US 308225
(43) Date of publication of application: 27.11.1991
(73) Proprietor: BIOSEARCH, INC., Plantation, Florida 33313 (US)
(72) Inventor: SILVESTRI, Loui J., Plantation, Florida 33313 (US); PYLE, Ruth H., Plantation, Florida 33313 (US)
(74) Representative: Werner, Hans-Karsten, Dr.Dipl.-Chem.
(86) International application number: PCT/US90/00750
(87) International publication number: WO 90/09166

(56) References cited:
- EP-A- 0 302 582
- EP-A- 0 333 523
- FR-A- 2 649 319
- US-A- 3 329 574
- US-A- 3 541 201

## Description

The invention relates to a multiphasic sustained release delivery system for prolonged, controlled delivery of a microencapsulated macromolecular bioactive agent.

US-A-3,329,574 and US-A-3,541,201 describe drug delivery compostiions for sustained release without using dosage forms with different releasing rates.

EP-A-0 302 582, a document according to Art. 54 (3) EPC, describes a drug delivery device, wherein peptides proteins etc. are encapsulated in two parts in such a way that the release of the drug of the second part begins when the release of the first parts ends. The object of the document is to provide a prolongated delivery of drugs at a constant dose.

EP-A-0 333 523, a document according to Art. 54 (3) EPC, describes method and compositions capable of delivering a bioactive agent to an animal entailing the steps of encapsulating effective amounts of the agent in a biocompatible excipient to form microcapsules having a size less than approximately ten micrometers, and administering effective amounts of the microcapsules to the animal.

In accordance with a first aspect of the present invention there is provided a multiphasic sustained release injectable delivery system for prolonged, controlled delivery of microencapsulated macromolecular bioactive agents of biological origin. The macromolecular bioactive agents include, but are not limited to: allergen-related substances such as Allergen source material, Allergen extracts, denatured IgE or fragments thereof, Pentapeptides, and IgE receptors or fragments thereof; cytokines such as Interferon, including alpha, gamma, etc., Interleukins, including Interleukins 1, 2, 3, 4, etc., Colony Stimulating Factors including macrophage colony stimulating factors and granulocyte colony stimulating factors, Epidermal Growth Factors, Fibroblast anti-collagenase, Erythropoietin, and Tumor Necrosis Factor; Anti-Hemophilic Factor (Factor VIII), and Receptor CD4. The system comprises the bioactive agent encapsulated in microcapsules of bioerodible encapsulating polymer, which permits a sustained, multiphasic release of said bioactive agent, including (i) a first portion of bioactive agent that upon injection is capable of being released from said microcapsules of bioerodible encapsulating polymer in a manner whereby only a relatively small amount of the bioactive agent is released during a first phase, whereby the initial biological reaction to the bioactive agent is minimized due to said first portion; and (ii) secondary portions of the bioactive agent that provide substantially higher levels of the bioactive agent in doses which could provoke a serious reaction in the patient, but for the prior release of said first portion. For example, a first portion of an allergen extract will produce a mild local reaction similar to that normally observed with low doses of conventional allergen administration.

The multiphasic sustained release delivery system of the invention permits a controlled release of the bioactive agent wherein the release profile in the treated patient describes an accelerating or crescendoing dosage delivery. The term crescendoing dosage delivery is defined herein to mean the release of the bioactive agents from the polymer matrix in doses that increase progressively over time, such that the rate of delivery of the bioactive agent at the end of the therapeutic period is much greater than the rate of delivery at the beginning of the therapeutic period. The rate of delivery at the end of the therapeutic period may either continue to increase until the matrices are exhausted of the bioactive substances, or the rate of delivery may plateau for a predetermined period of time at a rate high above the initial delivery rate before the matrices are exhausted of the bioactive substances.

The multiphasic sustained release delivery system of the invention may include one or more macromolecular bioactive agents of biological origin including but not limited to allergen-related substances such as Allergen source material, Allergen extracts, denatured IgE or fragments thereof, Pentapeptides, and IgE receptors or fragments thereof; cytokines such as Interferon, including alpha, gamma, etc., Interleukins including Interleukins 1, 2, 3, 4, etc., Colony Stimulating Factors including macrophage colony stimulating factors and granulocyte colony stimulating factors, Epidermal Growth Factors, Fibroblast anti-collagenase, Erythropoietin, and Tumor Necrosis Factor; Anti-Hemophilic Factor (Factor VIII), and Receptor CD4. The multiphasic sustained release delivery system may additionally include stabilizing agents including thermopreservatives such as glycerine or mannitol, adjuvant agents, and/or polymer hydrolysis modifying agents.

In a preferred embodiment, the bioactive agent is derived from naturally occurring sources. In another embodiment, the bioactive agent is genetically engineered or "cloned". In a further preferred embodiment, the bioactive agent is purified or partially purified. In a further preferred embodiment, the bioactive agent has been lyophilized. In another embodiment, stabilizing agents are used for the bioactive agent; preferred embodiments are thermopreservatives of which may be mentioned glycerine or mannitol.

The bioerodible encapsulating polymer is preferably chosen from a group of natural and synthetic polymers consisting of poly(lactides) and/or poly(glycolides) and/or copolymers and derivatives thereof; non-peptide polyamino acids; poly (ortho esters); low and high molecular weight polyanhydrides; polyiminocarbonates; poly alpha-aminoacids; polyalkyl-cyano-acrylate; polyphosphazenes; or acyloxymethyl polyaspartate and polyglutamate copolymers.

With the delivery system of the invention there is provided a method of therapy for living organisms, particularly humans and other mammals, which comprises injecting a subject with a microencapsulated macromolecular bioactive agent of biological origin including but not limited to: allergen-related substances such as Allergen source material, Allergen extracts, denatured IgE or fragments thereof, Pentapeptides, and IgE receptors or fragments thereof; cytokines such as Interferon, including alpha, gamma, etc., Interleukins including Interleukins 1, 2, 3, 4, etc., Colony Stimulating Factors including macrophage colony stimulating factors and granulocyte colony stimulating factors, Epidermal Growth Factors, Fibroblast anti-collagenase, Erythropoietin, and Tumor Necrosis Factor; Anti-Hemophilic Factor (Factor VIII), and Receptor CD4. The bioactive agent is encapsulated in microcapsules of bioerodible encapsulating polymer, which permits a sustained, multiphasic release of said bioactive agent, including (i) a first portion of which is capable of being released in a manner whereby only a relatively small amount of the bioactive agent is released during a first phase, whereby initial adverse reactivity is minimized due to said first portion producing a mild reaction similar to that normally observed with low doses of conventional bioactive agent administration; and (ii) secondary portions of the bioactive agent that provide substantially higher levels of the bioactive agent in doses which could provoke a serious reaction in the patient, but for the prior release of said first portion.

Furthermore, there is provided a method of allergen desensitization therapy which comprises injecting a subject with microencapsulated allergen extract. The allergen extract is encapsulated in microcapsules of bioerodible encapsulating polymer, which permits a sustained, multiphasic release of the allergen extract, including (i) a first portion of the allergen extract that upon injection is capable of being released in a manner whereby only a relatively small amount of the allergen extract is released during a first phase, whereby initial allergenicity is minimized due to said first portion producing a mild reaction similar to that normally observed with low doses of conventional allergen administration; and (ii) secondary portions of the allergen extract that provide substantially higher levels of allergen extract in doses which could provoke a serious reaction in the patient, but for the prior release of the first portion.

In accordance with yet another aspect of the present invention, there is provided a method of allergen desensitization therapy which comprises injecting a subject with microencapsulated unextracted allergen source material. The unextracted allergen source material is encapsulated in microcapsules, including (i) a first portion of said unextracted allergen source material that upon injection is capable of being exposed to body fluids and whose allergen content can thereafter be eluted in a manner whereby only a relatively small amount of allergen content is eluted during a first phase, whereby initial allergenicity is minimized due to said first portion producing a mild local reaction similar to that normally observed with low doses of conventional allergen administration; and (ii) secondary portions of unextracted allergen source material that provide substantially higher levels of allergen content in doses which could provoke a serious reaction in the patient, but for the prior release of said first portion.

The allergenic extract generally comprises one or more allergen sources including but not limited to the group of pollens, molds, foods, animal danders or their excretions, smuts and insects, their venoms or their excretions. In a preferred embodiment, the allergenic extract used is in agueous form. The allergens also may be physically or chemically modified. In a preferred embodiment, highly allergenic, low molecular weight allergen fractions have been excluded from the chemically modified allergenic extract. In a further preferred embodiment, the allergens extracts have been lyophilized. In another embodiment, the allergenic sources are not extracted at all, but rather are directly microencapsulated without prior extraction. In yet another embodiment, stabilizing agents are used for the allergen extract; preferred embodiments are thermopreservatives of which may be mentioned glycerine or mannitol.

In a preferred embodiment for any of the macromolecular bioactive agents, agents for symptomatic treatment of initial adverse reactions to the initial dose are included. Examples of these agents include: non-steroidal anti-inflammatory agents such as aspirin or ibuprofen; anti-histamines such as diphenhydramine, chlorpheniramine, clemastine, hydroxyzine, terfenadine promethazine, astemizole, loratadine, and the mast cell stabilizer cromolyn sodium; bronchodilators such as metaproterenol sulfate, isoetharine hydrochloride, theophylline, albuterol, and epinephrine; and corticosteroids such as prednisone, prednisolone, hydrocortisone, cortisone acetate, flunisolide, and triamincinolone acetate.

In a preferred embodiment for any of the macromolecular bioactive agents, an adjuvant is added such as tyrosine, polytyrosine, dimethylglycine (DMG), or muramyl dipeptide.

In a preferred embodiment for any of the macromolecular bioactive agents, a polymer hydrolysis modifying agent is included such as a non-toxic organic acid or base, or an acidic, neutral or basic inorganic salt, or a solution thereof.

In a preferred embodiment for any of the macromolecular bioactive agents, the variable release rate of the bioactive agent from the polymer is achieved heterogeneously by using a mixture of two or more of the following microsphere types:
1. Some types of microspheres have thinner bioactive agent-impregnated polymer layers, and others which have thicker bioactive agent-impregnated polymer layers.
2. Some types of microspheres which have higher concentrations of polymer, and others which have lower concentrations of polymer.
3. Some types of microspheres which have different ratios of copolymers which affect their rate of erosion.
4. Some types of microspheres which are made of one class of polymers, and others which are made of a different class of polymer with an intrinsically different means or rate of bioerosion.
5. Some types of microspheres which have higher concentrations of impregnated bioactive agent, and others which have lower concentrations of impregnated bioactive agent.

In a preferred embodiment, the variable release rate of the bioactive agent from the polymer is achieved homogeneously by using just one of the following types of microspheres:
1. Some types of microspheres which have thicker bioactive agent-impregnated outer polymer layers, and thinner bioactive agent impregnated polymer layers in the core.
2. Some types of microspheres in which there is a higher concentration of polymer in the outer layers of the microsphere, than in the core of the microsphere.
3. Some types of microspheres which have one ratio of copolymers in the outer layer but a different ratio of copolymer in the core of the microsphere.
4. Some types of microspheres in which the core of each microsphere is made of one class of polymers, and in which the outer layers are made of a different class of polymer with an intrinsically different means or rate of bioerosion.
5. Some types of microspheres in which the core of each microsphere contains a higher concentration of impregnated bioactive agent as compared to outer layers of each microsphere.

In a preferred embodiment, the variable release rate of the bioactive agent from the polymer is achieved through the use of a heterogeneous mixture of microspheres, some of which have hydrolysis accelerating agents impregnated in the microsphere polymer layers, while other microspheres contain hydrolysis retarding agents, or no accelerating/retarding agent at all. For example, the first portion (i) may include hydrolysis retarding agents impregnated in the microsphere polymer layers, while the microspheres corresponding to the secondary portions (ii) contain hydrolysis accelerating agents, or no accelerating agent at all. Alternatively, the first portion (i) may include hydrolysis retarding agents impregnated in the microsphere polymer layers, while the microspheres corresponding to said secondary portions (ii) contain hydrolysis accelerating agents.

### EXAMPLE 1

A heterogeneous microcapsule composition is provided wherein the final product is an admixture of one lot of microcapsules containing a 50:50 ratio of the excipients poly (lactide-co-glycolide) copolymer, and the second lot of microcapsules containing a 70:30 ratio of the excipients poly(lactide-co-glycolide) copolymer. The allergen is an aqueous extract of short ragweed (Ambrosia artemisifolia) present at approximately 0.4% (w/w), and no adjuvant or polymer hydrolysis-modifying agent is present. In two separate preparations, a total of 4.0 g of the excipient (in one case at a ratio of 50:50, and in the other 70:30) is dissolved in 200 g of methylene chloride. This solution is poured into a 250 ml round bottom flask fitted with a stainless steel swivel paddle and a light-duty, variable speed stirrer. A total volume of 1.5 ml of the allergen protein in buffered saline solution containing 10 mg/ml (as measured by the ninhydrin protein assay) is added to a 5 ml vial. The contents of the round bottomed flask are stirred at 3,000 rpm while the solution containing the allergen protein is slowly added. Alternatively, the mixture could be emulsified by ultrasonics and the like, or a high pressure homogenizer.

With continued stirring, 50 ml of silicone oil is added at the rate of 5.0 ml/minute using a peristaltic pump. The addition of the silicone oil results in the separation of the polymer phase and its subsequent deposit as droplets of solvent-swollen polymer onto the surface of the water-allergen microdroplets. These solvent-swollen polymer droplets are then coalesced to form a continuous film around the water-polypeptide microdroplets.

Hardening of the microcapsules is achieved by pouring the contents of the round bottom flask into approximately 1500 ml of heptane. This mixture is stirred at approximately 1000 rpm for 30 minutes using the same stainless steel stirrer. The liquid phase (containing heptane-methylene chloride-silicone oil) is removed by using a Buchner funnel loaded with Whatman #41 or #541 filter paper. The microcapsules are repeatedly washed with 100 ml aliquots of heptane to insure complete removal of the liquid phase, especially the silicone oil. Finally, the microcapsules are washed with deionized water followed by a wash with a 1% (w/v) solution of Tween 20 and dried at room temperature in vacuo. Microcapsules prepared in this fashion have diameters ranging from 5-50 microns.

### EXAMPLE 2

The procedure of Example 1 is repeated, except 50 mg/ml protein of the ragweed allergen extract is used. The resulting microspheres are then resuspended in 2.0 ml of an aqueous solution of ragweed allergen at only 10 mg protein/ml. Next, a total of 4.0 g of the excipient is dissolved in 200 g of methylene chloride, and this solution is poured into a 250 ml round bottom flask as described in Example 1. While rapidly stirring this mixture, the previously-prepared microspheres (now suspended in the aqueous solution of ragweed allergen) are slowly poured into the reaction flask. Immediately, 50 ml of silicone oil is added at the rate of 5.0 ml/minute. The product is processed as described in Example 1. The resulting product consists of microspheres containing ragweed allergen at 50 mg/ml, microspheres containing ragweed allergen at 10 mg/ml, and microspheres containing ragweed allergen with a core at 50 mg/ml and a "shell" at 10 mg ragweed protein/ml. The microspheres range in size from 5 to 400 », and are separated by mechanical sifting into various sizes. Dissolution studies demonstrate a desirable crescendoing multiphasic release profile for the larger (i.e., > 100 ») microspheres, thus indicating that the larger microspheres are predominantly hybrids.

### EXAMPLE 3

The procedure of Example 1 is repeated, except that Timothy grass (Phleum pratense) allergen is substituted for short ragweed, diketene acetal-diol condensates (diketene acetal 3,9-bis- [methylene]-2,4,8,10-tetraoxaspiro [5,5] undecane condensed with 1,6-hexanediol) is substituted for the (lactide-co-glycolide) copolymer, and an adjuvant (glycodipeptide N-acetyl-muramyl-L- alpha-aminobutyryl-D-isoglutamine) is incorporated into the aqueous phase.

### EXAMPLE 4

A total of 1.0 g of Poly(ortho ester) is dissolved in 25 ml methylene chloride. A total of 0.1 g of allergen protein from the house dust mite (Dermatophagoides farinae) is dissolved in phosphate buffered saline and 0.1% acetic acid. The microspheres are then prepared as described in Example 1.

### EXAMPLE 5

Example 4 is repeated, except that the house dust mite allergen has 0.1% sodium carbonate incorporated into the aqueous solution.

### EXAMPLE 6

The microspheres prepared by Example 4 ("acid" microspheres) are mixed with microspheres from Example 5 ("basic" microspheres) in a ratio of 1:1, 1:5, and 1:10 of "acid" to "base" microspheres, respectively. By preparing various mixtures of "acid" to "base" microspheres in this manner, the release profile of the product is dramatically changed. Specifically, the 1:1 mixture results in a slow initial release, and only a slightly elevated secondary release, whereas the 1:10 mixture results in an intermediate initial release, and a highly elevated secondary release.

### EXAMPLE 7

The procedure of Example 1 is repeated in substantial measure, except that a glutaraldehyde-modified ragweed allergen extract is used.

### EXAMPLE 8

The procedure of Example 1 is repeated, except an alum-adsorbed ragweed allergen extract is used.

### EXAMPLE 9

The procedure of Example 1 is followed, except microspheres are prepared with 5-60% glycerol incorporated into the aqueous phase as a thermopreservative for the ragweed allergen extract.

### EXAMPLE 10

This experiment is essentially the same as Example 1, except ragweed pollen instead of an aqueous ragweed extract is incorporated into the microspheres.

### EXAMPLE 11

This experiment is essentially the same as Example 1, except that lyophilized ragweed extract particles is first mechanically ground and sieved to produce a powder of approximately 10 » or smaller in size. The organic solvent is then loaded with these lyophilized allergen particles which is dispersed throughout the solution by stirring. Additional processing continues essentially as described in Example 1.

### EXAMPLE 12

This experiment is essentially the same as Example 2, except the reencapsulation is performed with an aqueous solution devoid of allergen. The final product consists of small microspheres without encapsulated allergen, small microspheres with the original concentration of allergen, and large microspheres that have an allergen core, but a thick encapsulating wall. These latter microspheres are appropriate in admixtures with thin-walled microsphere to produce the desired multi-linear release profile.

### EXAMPLE 13

A heterogeneous microcapsule composition is provided wherein the final product is an admixture of one lot of microcapsules containing a 50:50 ratio of the excipients poly(lactide-co-glycolide) copolymer, and the second lot of microcapsules containing a 70:30 ratio of the excipients poly(lactide-co-glycolide) copolymer. The bioactive agent is an aqueous solution of leukocyte-derived alpha interferon present at approximately 0.4% (w/w), and no adjuvant or polymer hydrolysis-modifying agent is present. In two separate preparations, a total of 4.0 g of the excipient (in one case at a ratio of 50:50, and in the other 70:30) is dissolved in 200 g of methylene chloride. This solution is poured into a 250 ml round bottom flask fitted with a stainless steel swivel paddle and a light-duty, variable speed stirrer. A total volume of 1.5 ml of the interferon protein in buffered saline solution containing 10 million units is added to the 5 ml vial. The contents of the round bottomed flask are stirred at 3,000 rpm while a solution containing the interferon protein is slowly added. Alternatively, the mixture could be emulsified by ultrasonics and the like, or a high pressure homogenizer.

With continued stirring, 50 ml of silicone oil is added at the rate of 5.0 ml/minute using a peristaltic pump. The addition of the silicone oil results in the separation of the polymer phase and its subsequent deposit as droplets of solvent-swollen polymer onto the surface of the water-allergen microdroplets. These solvent-swollen droplets are then coalesced to form a continuous film around the water-polypeptide microdroplets.

Hardening of the microcapsules is achieved by pouring the contents of the round bottom flask into approximately 1500 ml of heptane. This mixture is stirred at approximately 1000 rpm for 30 minutes using the same stainless steel stirrer. The liquid phase (containing heptane-methylene chloride-silicone oil) is removed by using a Buchner funnel loaded with Whatman #41 or #541 filter paper. The microcapsules are repeatedly washed with 100 ml aliquots of heptane to insure complete removal of the liquid phase, especially the silicone oil. Finally, the microcapsules are washed with deionized water followed by a wash with a 1% (w/v) solution of Tween 20 and dried at room temperature in vacuo. Microcapsules prepared in this fashion have diameters ranging from 5-50 microns.

### EXAMPLE 14

The procedure of Example 13 is repeated, except 100 million units of leukocyte-derived alpha interferon protein is used. The resulting microspheres are then resuspended in 2.0 ml of an aqueous solution of interferon protein at only 10 million units of interferon/ml. Next, a total of 4.0 g of the excipient is dissolved in 200 g of methylene chloride, and this solution is poured into a 250 ml round bottom flask as described in Example 13. While rapidly stirring this mixture, the previously-prepared microspheres (now suspended in the aqueous solution of interferon protein) are slowly poured into the reaction flask. Immediately, 50 ml of silicone oil is added at the rate of 5.0 ml/minute. The product is processed as described in Example 13. The resulting product consists of microspheres containing interferon at 100 million units/ml, microspheres containing interferon at 10 million units/ml, microspheres containing interferon with a core at 100 million units/ml, and a "shell" containing interferon at 10 million units/ml. The microspheres range in size from 5 to 400 », and are separated by mechanical sifting into various sizes. Dissolution studies demonstrate a desirable crescendoing multiphasic release profile for the larger (i.e., > 100 ») microspheres, thus indicating that the larger microspheres are predominantly hybrids.

## Claims (Claims for the following Contracting State(s): AT, BE, CH, LI, DE, DK, FR, GB, IT, LU, NL, SE)

1. A multiphasic sustained release delivery system for prolonged, controlled delivery of microencapsulated macromolecular bioactive agent, comprising a macromolecular bioactive agent of a biological origin encapsulated in microcapsules of bioerodible encapsulating polymer, which permits a sustained, multiphasic release of said macromolecular bioactive agent, including (i) a first portion of said macromolecular bioactive agent that upon injection is capable of being released from said microcapsules of bioerodible encapsulating polymer in a manner whereby only a relatively small amount of said macromolecular bioactive agent is released during a first phase, whereby initial adverse biological reaction is minimized due to said first portion producing a mild reaction similar to that normally observed with low doses of conventional administration; and (ii) secondary portions of said macromolecular bioactive agent to provide a substantially higher level of the macromolecular bioactive agent in doses which could provoke a serious reaction in the patient, but for the prior release of said first portion.

2. A multiphasic sustained release delivery system according to claim 1 wherein the macromolecular bioactive agent is selected from the group consisting of allergen-related substances, cytokines, anti-hemophilic factors and Receptor CD4.

3. A multiphasic sustained release delivery system according to claim 1 wherein the first portion and secondary portions of said macromolecular bioactive agent provide a controlled release providing a dosage profile having an accelerating or crescendoing release.

4. A multiphasic sustained release injectable delivery system for prolonged, controlled delivery of microencapsulated macromolecular bioactive agent in accordance with claim 1, wherein the harmful effects of the released macromolecular bioactive agents are controlled by the synchronous release of an agent for symptomatic treatment of initial adverse reactions to the initial dose.

5. A multiphasic sustained release injectable delivery system of claim 4 wherein said agent for symptomatic treatment comprises a non-steroidal anti-inflammatory agent, an anti-histamine, a corticosteroid, or a bronchodilator.

6. A multiphasic sustained release injectable delivery system for prolonged, controlled delivery of microencapsulated macromolecular bioactive agent of claim 1, wherein said bioerodible encapsulating polymer is a member selected from the group of poly(lactides), poly(glycolides), and copolymers and derivatives thereof; non-peptide polyamino acids; poly (ortho esters); low and high molecular weight polyanhydrides; polyiminocarbonates; poly alpha-aminoacids; polyalkyl-cyano-acrylate; polyphosphazenes; and acyloxymethyl polyaspartate and polyglutamate copolymers.

7. A multiphasic sustained release injectable delivery system for prolonged, controlled delivery of microencapsulated bioactive agent of claim 1, wherein the multiphasic release rate is achieved by providing a heterogeneous mixture of microspheres of bioerodible encapsulating polymers, a portion of said microspheres corresponding to the first portion (i) having a combination of one or more of the following:
a. thinner bioactive agent-impregnated polymer layers,
b. higher concentrations of polymer,
c. lower concentrations of impregnated bioactive agent;
and a portion of said microspheres corresponding to said secondary portions (ii) having a combination of one or more of the following:
a. thicker bioactive agent impregnated polymer layers,
b. lower concentrations of polymer,
c. different ratios of copolymers which affect their rate of erosion as compared to the first portion,
d. different class of polymer with an intrinsically different means or rate of bioerosion as compared to the first portion,
e. higher concentrations of impregnated bioactive agent.

8. A multiphasic sustained release injectable delivery system for prolonged, controlled delivery of microencapsulated bioactive agent of claim 1, wherein the multiphasic release rate is homogeneously achieved by using only one of the following types of microspheres :
a. microspheres which have thicker bioactive agent-impregnated outer polymer layers, and thinner bioactive agent impregnated polymer layers in the core, or
b. microspheres in which there is a higher concentration of polymer in the outer layers of the microsphere, than in the core of the microsphere, or
c. microspheres which have different ratios of copolymers in the outer layers as compared to the core, or
d. microspheres in which the outer layer is made of one class of polymer, and in which the core of each microsphere is made of a different class of polymer with an intrinsically different means or rate of bioerosion, or
e. microspheres in which the outer layer of each microsphere contains a lower concentration of impregnated bioactive agent as compared to the core of each microsphere whereby the bioactive agent of the first portion (i) is contained in the outer layers and the bioactive agent of the secondary portions (ii) is contained in the core.

9. A multiphasic sustained release injectable delivery system for prolonged, controlled delivery of microencapsulated bioactive agent of claim 1, wherein the multiphasic release rate is achieved by providing a mixture of microspheres, those corresponding to said first portion (i) including hydrolysis retarding agents impregnated in the microsphere polymer layers, while the microspheres corresponding to said secondary portions (ii) contain hydrolysis accelerating agents, or no accelerating agent at all.

10. A multiphasic sustained release injectable delivery system of claim 9 wherein said hydrolysis retarding and accelerating agents are non-toxic organic acids or bases, or an acidic, neutral or basic inorganic salt, or a solution thereof.

11. A multiphasic sustained release injectable delivery system for prolonged, controlled delivery of microencapsulated bioactive agent of claim 1, wherein the multiphasic release rate is achieved by providing a mixture of microspheres, those corresponding to said first portion (i) may include no hydrolysis retarding agents impregnated in the microsphere polymer layers, while the microspheres corresponding to said secondary portions (ii) contain hydrolysis accelerating agents.

12. A multiphasic sustained release injectable delivery system of claim 11 wherein said hydrolysis retarding and accelerating agents are non-toxic organic acids or bases, or an acidic, neutral or basic inorganic salt, or a solution thereof.

13. A multiphasic sustained release injectable delivery system of claim 1 wherein a stabilizing agent is encapsulated along with the bioactive agent.

14. A multiphasic sustained release injectable delivery system of claim 13 wherein said stabilizing agent is the thermopreservative glycerine or mannitol.

15. A multiphasic sustained release injectable delivery system of claim 1 wherein an adjuvant is encapsulated along with the bioactive agent.

16. A multiphasic sustained release injectable delivery system of claim 15 wherein said adjuvant is tyrosine, polytyrosine, dimethylglycine (DMG), or muramyl dipeptide.

17. A multiphasic sustained release injectable delivery system of claim 1 wherein the encapsulated bioactve agent is in aqueous form.

18. A multiphasic sustained release injectable delivery system of claim 1 wherein the encapsulated bioactive agent is in lyophilized form.

19. A multiphasic sustained release injectable delivery system of claim 1 wherein the macromolecular bioactive agent is unextracted allergen source material.

20. A multiphasic sustained release injectable delivery system for prolonged, controlled delivery of microencapsulated allergen extract comprising allergen extract encapsulated in microcapsules of bioerodible encapsulating polymer, which permits a sustained, multiphasic release of said allergen extract, including (i) a first portion of allergen extract that upon injection is capable of being released from said microcapsules of bioerodible encapsulating polymer in a manner whereby only a relatively small amount of said allergen extract is released during a first phase, whereby initial allergenicity is minimized due to said first portion producing a mild local reaction similar to that normally observed with low doses of conventional allergen administration; and (ii) secondary portions of allergen extract that provide a substantially higher level of allergen extracts in doses which could provoke a serious reaction in the patient, but for the prior releases of said first portion.

21. A multiphasic sustained injectable delivery system for prolonged, controlled delivery of microencapsulated allergen extract in accordance with claim 20, wherein the total amount of allergen extract in said secondary portions are at levels higher than would be a toxic level for said allergen extract, in the absence of the prior release of said first portion.

22. A multiphasic sustained release injectable delivery system for prolonged, controlled delivery of microencapsulated allergen extract in accordance with claim 20, wherein the harmful effects of the released allergens are controlled by the synchronous release of an agent for symptomatic treatment of initial adverse reactions to the initial dose.

23. A multiphasic sustained release injectable delivery system of claim 22 wherein said agent comprises a non-steroidal anti-inflammatory agent, an anti-histamine, a corticosteroid, or a bronchodilator.

24. A multiphasic sustained release injectable delivery system for prolonged, controlled delivery of microencapsulated allergen extract of claim 20, wherein said bioerodible encapsulating polymer is a member selected from the group of poly(lactides), poly(glycolides), and copolymers and derivatives thereof; non-peptide polyamino acids; poly (ortho esters); low and high molecular weight polyanhydrides; polyiminocarbonates; poly alpha-aminoacids; polyalkyl-cyano-acrylate; polyphosphazenes; and acyloxymethyl polyaspartate and polyglutamate copolymers.

25. A multiphasic sustained release injectable delivery system for prolonged, controlled delivery of microencapsulated allergen extract of claim 20, wherein said allergen extract is of an allergen source selected from the group consisting of a pollen, mold, food, animal dander, animal excretion, smut, insect, insect venom and insect excretions.

26. A multiphasic sustained release injectable delivery system for prolonged, controlled delivery of microencapsulated allergen extract of claim 20, wherein the multiphasic release rate is achieved by providing a heterogeneous mixture of microspheres of bioerodible encapsulating polymers, a portion of said microspheres corresponding to the first portion (i) having a combination of one or more of the following:
a. thinner allergen-impregnated polymer layers,
b. higher concentrations of polymer,
c. lower concentrations of impregnated allergen;
and a portion of said microspheres corresponding to said secondary portions (ii) having a combination of one or bore of the following:
a. thicker allergen impregnated polymer layers,
b. lower concentrations of polymer,
c. different ratios of copolymers which affect their rate of erosion as compared to the first portion,
d. different class of polymer with an intrinsically different means or rate of bioerosion as compared to the first portion,
e. higher concentrations of impregnated allergen.

27. A multiphasic sustained release injectable delivery system for prolonged, controlled delivery of microencapsulated allergen extract of claim 20, wherein the multiphasic release rate is homogeneously achieved by using only one of the following types of microspheres :
a. microspheres which have thicker allergen-impregnated outer polymer layers, and thinner allergen impregnated polymer layers in the core, or
b. microspheres in which there is a higher concentration of polymer in the outer layers of the microsphere, than in the core of the microsphere, or
c. microspheres which have different ratios of copolymers in the outer layers as compared to the core, or
d. microspheres in which the outer layer is made of one class of polymer, and in which the core of each microsphere is made of a different class of polymer with an intrinsically different means or rate of bioerosion, or
e. microspheres in which the outer layer of each microsphere contains a lower concentration of impregnated allergen as compared to the core of each microsphere whereby the allergen extract of the first portion (i) is contained in the outer layers and the allergen extract of the secondary portions (ii) is contained in the core.

28. A multiphasic sustained release injectable delivery system for prolonged, controlled delivery of microencapsulated allergen extract of claim 20, wherein the multiphasic release rate is achieved by providing a mixture of microspheres, those corresponding to said first portion (i) including hydrolysis retarding agents impregnated in the microsphere polymer layers, while the microspheres corresponding to said secondary portions (ii) contain hydrolysis accelerating agents, or no accelerating agent at all.

29. A multiphasic sustained release injectable delivery system of claim 28 wherein said hydrolysis retarding and accelerating agents are non-toxic organic acids or bases, or an acidic, neutral or basic inorganic salt, or a solution thereof.

30. A multiphasic sustained release injectable delivery system for prolonged, controlled delivery of microencapsulated allergen extract of claim 20, wherein the multiphasic release rate is achieved by providing a mixture of microspheres, those corresponding to said first portion (i) may include no hydrolysis retarding agents impregnated in the microsphere polymer layers, while the microspheres corresponding to said secondary portions (ii) contain hydrolysis accelerating agents.

31. A multiphasic sustained release injectable delivery system of claim 30 wherein said hydrolysis retarding and accelerating agents are non-toxic organic acids or bases, or an acidic, neutral or basic inorganic salt, or a solution thereof.

32. A multiphasic sustained release injectable delivery system of claim 20 wherein an allergen stabilizing agent is encapsulated along with the allergen extract.

33. A multiphasic sustained release injectable delivery system of claim 32 wherein said allergen stabilizing agent is the thermopreservative glycerine or mannitol.

34. A multiphasic sustained release injectable delivery system of claim 20 wherein an adjuvant is encapsulated along with the allergen extract.

35. A multiphasic sustained release injectable delivery system of claim 34 wherein said adjuvant is tyrosine, polytyrosine, dimethylglycine (DMG), or muramyl dipeptide.

36. A multiphasic sustained release injectable delivery system of claim 20 wherein the encapsulated allergen extract is in aqueous form.

37. A multiphasic sustained release injectable delivery system of claim 20 wherein the encapsulated allergen is in lyophilized form.

38. A multiphasic sustained release injectable delivery system of claim 20 wherein the encapsulated allergen extract is physically or chemically modified.

39. A multiphasic sustained release injectable delivery system of claim 38 wherein low molecular weight allergen fractions have been excluded from said physically or chemically modified allergens.

## Claims (Claims for the following Contracting State(s): ES)

1. A multiphasic sustained release delivery system for prolonged, controlled delivery of microencapsulated macromolecular bioactive agent, comprising a macromolecular bioactive agent of a biological origin encapsulated in microcapsules of bioerodible encapsulating polymer, which permits a sustained, multiphasic release of said macromolecular bioactive agent, including (i) a first portion of said macromolecular bioactive agent that upon injection is capable of being released from said microcapsules of bioerodible encapsulating polymer in a manner whereby only a relatively small amount of said macromolecular bioactive agent is released during a first phase, whereby initial adverse biological reaction is minimized due to said fist portion producing a mild reaction similar to that normally observed with low doses of conventional administration; and (ii) secondary portions of said macromolecular bioactive agent to provide a substantially higher level of the macromolecular bioactive agent in doses which could provoke a serious reaction in the patient, but for the prior release of said first portion.

2. A multiphasic sustained release delivery system according to claim 1 wherein the macromolecular bioactive agent is selected from the group consisting of allergen-related substances, cytokines, anti-hemophilic factors and Receptor CD4.

3. A multiphasic sustained release delivery system according to claim 1 wherein the first portion and secondary portions of said macromolecular bioactive agent provide a controlled release providing a dosage profile having an accelerating or crescendoing release.

4. A multiphasic sustained release injectable delivery system for prolonged, controlled delivery of microencapsulated macromolecular bioactive agent in accordance with claim 1, wherein the harmful effects of the released macromolecular bioactive agents are controlled by the synchronous release of an agent for symptomatic treatment of initial adverse reactions to the initial dose.

5. A multiphasic sustained release injectable delivery system of claim 4 wherein said agent for symptomatic treatment comprises a non-steroidal anti-inflammatory agent, an anti-histamine, a corticosteroid, or a bronchodilator.

6. A multiphasic sustained release injectable delivery system for prolonged, controlled delivery of microencapsulated macromolecular bioactive agent of claim 1, wherein said bioerodible encapsulating polymer is a member selected from the group of poly(lactides), poly(glycolides), and copolymers and derivatives thereof; non-peptide polyamino acids; poly (ortho esters); low and high molecular weight polyanhydrides; polyiminocarbonates; poly alpha-aminoacids; polyalkyl-cyano-acrylate; polyphosphazenes; and acyloxymethyl polyaspartate and polyglutamate copolymers.

7. A multiphasic sustained release injectable delivery system for prolonged, controlled delivery of microencapsulated bioactive agent of claim 1, wherein the multiphasic release rate is achieved by providing a heterogeneous mixture of microspheres of bioerodible encapsulating polymers, a portion of said microspheres corresponding to the first portion (i) having a combination of one or more of the following:
a. thinner bioactive agent-impregnated polymer layers,
b. higher concentrations of polymer,
c. lower concentrations of impregnated bioactive agent;
and a portion of said microspheres corresponding to said secondary portions (ii) having a combination of one or more of the following:
a. thicker bioactive agent impregnated polymer layers,
b. lower concentrations of polymer,
c. different ratios of copolymers which affect their rate of erosion as compared to the first portion,
d. different class of polymer with an intrinsically different means or rate of bioerosion as compared to the first portion,
e. higher concentrations of impregnated bioactive agent.

8. A multiphasic sustained release injectable delivery system for prolonged, controlled delivery of microencapsulated bioactive agent of claim 1, wherein the multiphasic release rate is homogeneously achieved by using only one of the following types of microspheres :
a. microspheres which have thicker bioactive agent-impregnated outer polymer layers, and thinner bioactive agent impregnated polymer layers in the core, or
b. microspheres in which there is a higher concentration of polymer in the outer layers of the microsphere, than in the core of the microsphere, or
c. microspheres which have different ratios of copolymers in the outer layers as compared to the core, or
d. microspheres in which the outer layer is made of one class of polymer, and in which the core of each microsphere is made of a different class of polymer with an intrinsically different means or rate of bioerosion, or
e. microspheres in which the outer layer of each microsphere contains a lower concentration of impregnated bioactive agent as compared to the core of each microsphere whereby the bioactive agent of the first portion (i) is contained in the outer layers and the bioactive agent of the secondary portions (ii) is contained in the core.

9. A multiphasic sustained release injectable delivery system for prolonged, controlled delivery of microencapsulated bioactive agent of claim 1, wherein the multiphasic release rate is achieved by providing a mixture of microspheres, those corresponding to said first portion (i) including hydrolysis retarding agents impregnated in the microsphere polymer layers, while the microspheres corresponding to said secondary portions (ii) contain hydrolysis accelerating agents, or no accelerating agent at all.

10. A multiphasic sustained release injectable delivery system of claim 9 wherein said hydrolysis retarding and accelerating agents are non-toxic organic acids or bases, or an acidic, neutral or basic inorganic salt, or a solution thereof.

11. A multiphasic sustained release injectable delivery system for prolonged, controlled delivery of microencapsulated bioactive agent of claim 1, wherein the multiphasic release rate is achieved by providing a mixture of microspheres, those corresponding to said first portion (i) may include no hydrolysis retarding agents impregnated in the microsphere polymer layers, while the microspheres corresponding to said secondary portions (ii) contain hydrolysis accelerating agents.

12. A multiphasic sustained release injectable delivery system of claim 11 wherein said hydrolysis retarding and accelerating agents are non-toxic organic acids or bases, or an acidic, neutral or basic inorganic salt, or a solution thereof.

13. A multiphasic sustained release injectable delivery system of claim 1 wherein a stabilizing agent is encapsulated along with the bioactive agent.

14. A multiphasic sustained release injectable delivery system of claim 13 wherein said stabilizing agent is the thermopreservative glycerine or mannitol.

15. A multiphasic sustained release injectable delivery system of claim 1 wherein an adjuvant is encapsulated along with the bioactive agent.

16. A multiphasic sustained release injectable delivery system of claim 15 wherein said adjuvant is tyrosine, polytyrosine, dimethylglycine (DMG), or muramyl dipeptide.

17. A multiphasic sustained release injectable delivery system of claim 1 wherein the encapsulated bioactive agent is in aqueous form.

18. A multiphasic sustained release injectable delivery system of claim 1 wherein the encapsulated bioactive agent is in lyophilized form.

19. A multiphasic sustained release injectable delivery system of claim 1 wherein the macromolecular bioactive agent is unextracted allergen source material.

20. A multiphasic sustained release injectable delivery system for prolonged, controlled delivery of microencapsulated allergen extract comprising allergen extract encapsulated in microcapsules of bioerodible encapsulating polymer, which permits a sustained, multiphasic release of said allergen extract, including (i) a first portion of allergen extract that upon injection is capable of being released from said microcapsules of bioerodible encapsulating polymer in a manner whereby only a relatively small amount of said allergen extract is released during a first phase, whereby initial allergenicity is minimized due to said first portion producing a mild local reaction similar to that normally observed with low doses of conventional allergen administration; and (ii) secondary portions of allergen extract that provide a substantially higher level of allergen extracts in doses which could provoke a serious reaction in the patient, but for the prior releases of said first portion.

21. A multiphasic sustained injectable delivery system for prolonged, controlled delivery of microencapsulated allergen extract in accordance with claim 20, wherein the total amount of allergen extract in said secondary portions are at levels higher than would be a toxic level for said allergen extract, in the absence of the prior release of said first portion.

22. A multiphasic sustained release injectable delivery system for prolonged, controlled delivery of microencapsulated allergen extract in accordance with claim 20, wherein the harmful effects of the released allergens are controlled by the synchronous release of an agent for symptomatic treatment of initial adverse reactions to the initial dose.

23. A multiphasic sustained release injectable delivery system of claim 22 wherein said agent comprises a non-steroidal anti-inflammatory agent, an anti-histamine, a corticosteroid, or a bronchodilator.

24. A multiphasic sustained release injectable delivery system for prolonged, controlled delivery of microencapsulated allergen extract of claim 20, wherein said bioerodible encapsulating polymer is a member selected from the group of poly(lactides), poly(glycolides), and copolymers and derivatives thereof; non-peptide polyamino acids; poly (ortho esters); low and high molecular weight polyanhydrides; polyiminocarbonates; poly alpha-aminoacids; polyalkyl-cyano-acrylate; polyphosphazenes; and acyloxymethyl polyaspartate and polyglutamate copolymers.

25. A multiphasic sustained release injectable delivery system for prolonged, controlled delivery of microencapsulated allergen extract of claim 20, wherein said allergen extract is of an allergen source selected from the group consisting of a pollen, mold, food, animal dander, animal excretion, smut, insect, insect venom and insect excretions.

26. A multiphasic sustained release injectable delivery. system for prolonged, controlled delivery of microencapsulated allergen extract of claim 20, wherein the multiphasic release rate is achieved by providing a heterogeneous mixture of microspheres of bioerodible encapsulating polymers, a portion of said microspheres corresponding to the first portion (i) having a combination of one or more of the following:
a. thinner allergen-impregnated polymer layers,
b. higher concentrations of polymer,
c. lower concentrations of impregnated allergen;
and a portion of said microspheres corresponding to said secondary portions (ii) having a combination of one or more of the following:
a. thicker allergen impregnated polymer layers,
b. lower concentrations of polymer,
c. different ratios of copolymers which affect their rate of erosion as compared to the first portion,
d. different class of polymer with an intrinsically different means or rate of bioerosion as compared to the first portion,
e. higher concentrations of impregnated allergen.

27. A multiphasic sustained release injectable delivery system for prolonged, controlled delivery of microencapsulated allergen extract of claim 20, wherein the multiphasic release rate is homogeneously achieved by using only one of the following types of microspheres :
a. microspheres which have thicker allergen-impregnated outer polymer layers, and thinner allergen impregnated polymer layers in the core, or
b. microspheres in which there is a higher concentration of polymer in the outer layers of the microsphere, than in the core of the microsphere, or
c. microspheres which have different ratios of copolymers in the outer layers as compared to the core, or
d. microspheres in which the outer layer is made of one class of polymer, and in which the core of each microsphere is made of a different class of polymer with an intrinsically different means or rate of bioerosion, or
e. microspheres in which the outer layer of each microsphere contains a lower concentration of impregnated allergen as compared to the core of each microsphere whereby the allergen extract of the first portion (i) is contained in the outer layers and the allergen extract of the secondary portions (ii) is contained in the core.

28. A multiphasic sustained release injectable delivery system for prolonged, controlled delivery of microencapsulated allergen extract of claim 20, wherein the multiphasic release rate is achieved by providing a mixture of microspheres, those corresponding to said first portion (i) including hydrolysis retarding agents impregnated in the microsphere polymer layers, while the microspheres corresponding to said secondary portions (ii) contain hydrolysis accelerating agents, or no accelerating agent at all.

29. A multiphasic sustained release injectable delivery system of claim 28 wherein said hydrolysis retarding and accelerating agents are non-toxic organic acids or bases, or an acidic, neutral or basic inorganic salt, or a solution thereof.

30. A multiphasic sustained release injectable delivery system for prolonged, controlled delivery of microencapsulated allergen extract of claim 20, wherein the multiphasic release rate is achieved by providing a mixture of microspheres, those corresponding to said first portion (i) may include no hydrolysis retarding agents impregnated in the microsphere polymer layers, while the microspheres corresponding to said secondary portions (ii) contain hydrolysis accelerating agents.

31. A multiphasic sustained release injectable delivery system of claim 30 wherein said hydrolysis retarding and accelerating agents are non-toxic organic acids or bases, or an acidic, neutral or basic inorganic salt, or a solution thereof.

32. A multiphasic sustained release injectable delivery system of claim 20 wherein an allergen stabilizing agent is encapsulated along with the allergen extract.

33. A multiphasic sustained release injectable delivery system of claim 32 wherein said allergen stabilizing agent is the thermopreservative glycerine or mannitol.

34. A multiphasic sustained release injectable delivery system of claim 20 wherein an adjuvant is encapsulated along with the allergen extract.

35. A multiphasic sustained release injectable delivery system of claim 34 wherein said adjuvant is tyrosine, polytyrosine, dimethylglycine (DMG), or muramyl dipeptide.

36. A multiphasic sustained release injectable delivery system of claim 20 wherein the encapsulated allergen extract is in aqueous form.

37. A multiphasic sustained release injectable delivery system of claim 20 wherein the encapsulated allergen is in lyophilized form.

38. A multiphasic sustained release injectable delivery system of claim 20 wherein the encapsulated allergen extract is physically or chemically modified.

39. A multiphasic sustained release injectable delivery system of claim 38 wherein low molecular weight allergen fractions have been excluded from said physically or chemically modified allergens.

40. Process for preparing a sustained release dosage form by encapsulating the bioactive agent of claims 1 to 39 in microcapsules of bioerodible encapsulating polymer.

## Patentansprüche (Patentansprüche für folgende(n) Vertragsstaat(en): AT, BE, CH, LI, DE, DK, FR, GB, IT, LU, NL, SE)

1. Mehrphasiges Abgabesystem mit Langzeitfreisetzung für längere, kontrollierte Abgabe eines mikroverkapselten makromolekularen bioaktiven Mittels, das ein makromolekulares bioaktives Mittel biologischen Ursprungs umfaßt, eingekapselt in Mikrokapseln aus bioerodierbarem verkapselnden Polymer, das eine anhaltende, mehrphasige Freisetzung des makromolekularen bioaktiven Mittels gestattet, umfassend
(i) einen ersten Teil des makromolekularen bioaktiven Mittels, der nach Injektion aus den Mikrokapseln aus bioerodierbarem verkapselnden Polymer in einer Weise freisetzbar ist, daß nur eine relativ kleine Menge des makromolekularen bioaktiven Mittels im Laufe einer ersten Phase freigesetzt wird, wobei die einsetzende ungünstige biologische Reaktion aufgrund dessen minimiert wird, daß der erste Teil eine milde Reaktion erzeugt, ähnlich der, die normalerweise bei niedrigen Dosen einer herkömmlichen Verabreichung beobachtet wird; und
(ii) weitere Mengen des makromolekularen bioaktiven Mittels, um eine wesentlich höhere Konzentration an makromolekularem bioaktiven Mittel in Dosen zu ergeben, die ohne die vorherige Freisetzung des ersten Teils eine ernstliche Reaktion am Patienten hervorrufen könnten.

2. Mehrphasiges Abgabesystem mit Langzeitfreisetzung nach Anspruch 1, wobei das makromolekulare bioaktive Mittel ausgewählt ist aus der Gruppe bestehend aus Allergenverwandten Substanzen, Cytokinen, Antihämophiliefaktoren und Rezeptor CD4.

3. Mehrphasiges Abgabesystem mit Langzeitfreisetzung nach Anspruch 1, wobei erster Teil und weitere Mengen des makromolekularen bioaktiven Mittels eine kontrollierte Freisetzung bereitstellen, die ein Dosierungsprofil mit beschleunigender oder zunehmender Freisetzung ergeben.

4. Mehrphasiges injizierbares Abgabesystem mit Langzeitfreisetzung für längere, kontrollierte Abgabe eines mikroverkapselten makromolekularen bioaktiven Mittels nach Anspruch 1, wobei die schädlichen Wirkungen des makromolekularen bioaktiven Mittels kontrolliert werden durch die synchrone Freisetzung eines Mittels zur symptomatischen Behandlung einsetzender nachteiliger Wirkungen der anfänglichen Dosis.

5. Mehrphasiges injizierbares Abgabesystem mit Langzeitfreisetzung nach Anspruch 4, wobei das Mittel zur symptomatischen Behandlung ein entzündungshemmendes nichtsteroides Mittel, ein Antihistaminikum, ein Corticosteroid oder einen Bronchodilatator umfaßt.

6. Mehrphasiges injizierbares Abgabesystem mit Langzeitfreisetzung für längere, kontrollierte Abgabe eines mikroverkapselten makromolekularen bioaktiven Mittels nach Anspruch 1, wobei das bioerodierbare verkapselnde Polymer ein Vertreter ist, ausgewählt aus der Gruppe der Polylactide, Polyglycolide und deren Copolymeren und Derivaten; Nichtpeptid-Polyaminosäuren; Polyorthoester; nieder- und hochmolekularen Polyanhydride; Polyiminocarbonate; Poly-α-aminosäuren; Polyalkylcyanacrylate; Polyphosphazene; sowie Acyloxymethylpolyaspartate und Polyglutamat-Copolymere.

7. Mehrphasiges injizierbares Abgabesystem mit Langzeitfreisetzung für längere, kontrollierte Abgabe eines mikroverkapselten bioaktiven Mittels nach Anspruch 1, wobei die Geschwindigkeit der mehrphasigen Freisetzung erreicht wird durch Bereitstellen einer heterogenen Mischung aus Mikrokugeln bioerodierbarer verkapselnder Polymere, wobei ein Teil der dem ersten Teil (i) entsprechenden Mikrokugeln eine Kombination aus einem oder mehreren der folgenden aufweist:
a. dünnere, mit bioaktivem Mittel imprägnierte Polymer-Schichten;
b. höhere Konzentrationen an Polymer;
c. niedrigere Konzentrationen an imprägniertem bioaktivem Mittel;
und ein Teil der den weiteren Mengen (ii) entsprechenden Mikrokugeln eine Kombination aus einem oder mehreren der folgenden aufweist:
a. dickere, mit bioaktivem Mittel imprägnierte Polymer-Schichten;
b. niedrigere Konzentrationen an Polymer;
c. unterschiedliche Verhältnisse an Copolymeren, die deren Erosionsgeschwindigkeit im Vergleich zum ersten Teil beeinflussen;
d. eine andere Polymerklasse mit einer ihr eigenen anderen Erosionsart oder -geschwindigkeit im Vergleich zum ersten Teil;
e. höhere Konzentrationen an imprägniertem bioaktiven Mittel.

8. Mehrphasiges injizierbares Abgabesystemmit Langzeitfreisetzung für längere, kontrollierte Abgabe eines mikroverkapselten Mittels nach Anspruch 1, wobei die Geschwindigkeit der mehrphasigen Freisetzung homogen erreicht wird durch Verwendung nur einer der folgenden Art Mikrokugeln:
a. Mikrokugeln, die dickere, mit bioaktivem Mittel imprägnierte äußere Polymer-Schichten aufweisen und dünnere, mit bioaktivem Mittel imprägnierte Polymer-Schichten im Kern; oder
b. Mikrokugeln, bei denen eine höhere Konzentration an Polymer in der äußeren Schichten der Mikrokugel als im Kern der Mikrokugel vorliegt; oder
c. Mikrokugeln, die unterschiedliche Verhältnisse an Copolymeren in den äußeren Schichten im Vergleich zum Kern aufweisen; oder
d. Mikrokugeln, bei denen die äußere Schicht aus einer Polymerklasse ist, und bei denen der Kern einer jeden Mikrokugel aus einer anderen Polymerklasse mit einer ihr eigenen anderen Erosionsart oder -geschwindigkeit ist; oder
e. Mikrokugeln, bei denen die äußere Schicht einer jeden Mikrokugel im Vergleich zum Kern einer jeden Mikrokugel eine niedrigere Konzentration an imprägniertem bioaktiven Mittel enthält, wodurch das bioaktive Mittel des ersten Teils (i) in den äußeren Schichten enthalten ist, und das bioaktive Mittel der weiteren Mengen (ii) im Kern enthalten ist.

9. Mehrphasiges injizierbares Abgabesystem mit Langzeitfreisetzung für längere, kontrollierte Abgabe eines mikroverkapselten bioaktiven Mittels nach Anspruch 1, wobei die Geschwindigkeit der mehrphasigen Freisetzung erzielt wird durch Bereitstellen einer Mischung aus Mikrokugeln, wobei die dem ersten Teil (i) entsprechenden hydrolyseverzögernde Mittel in den Mikrokugel-Polymerschichten imprägniert umfassen, während die den weiteren Mengen (ii) entsprechenden Mikrokugeln hydrolysebeschleunigende Mittel oder gar keine beschleunigenden Mittel enthalten.

10. Mehrphasiges injizierbares Abgabesystemmit Langzeitfreisetzung nach Anspruch 9, wobei die hydrolyseverzögernden und -beschleunigenden Mittel nichttoxische organische Säuren oder Basen sind, oder ein saures, neutrales oder basisches anorganisches Salz oder eine Lösung derselben.

11. Mehrphasiges injizierbares Abgabesystem mit Langzeitfreisetzung für längere, kontrollierte Abgabe eines mikroverkapselten bioaktiven Mittels nach Anspruch 1, wobei die Geschwindigkeit der mehrphasigen Freisetzung erzielt wird durch Bereitstellen einer Mischung aus Mikrokugeln, wobei die dem ersten Teil (i) entsprechenden keine hydrolyseverzögernden Mittel in den Mikrokugel-Polymerschichten imprägniert enthalten können, während die den weiteren Mengen (ii) entsprechenden Mikrokugeln hydrolysebeschleunigende Mittel enthalten.

12. Mehrphasiges injizierbares Abgabesystemmit Langzeitfreisetzung nach Anspruch 1, wobei die hydrolyseverzögernden und -beschleunigenden Mittel nichttoxische organische Säuren oder Basen sind, oder ein saures, neutrales oder basisches anorganisches Salz oder eine Lösung derselben.

13. Mehrphasiges injizierbares Abgabesystem mit Langzeitfreisetzung nach Anspruch 1, wobei neben dem bioaktiven Mittel ein Stabilisierungsmittel eingekapselt ist.

14. Mehrphasiges injizierbares Abgabesystem mit Langzeitfreisetzung nach Anspruch 13, wobei das Stabilisierungsmittel das Thermokonservierungsmittel Glycerin oder Mannit ist.

15. Mehrphasiges injizierbares Abgabesystem mit Langzeitfreisetzung nach Anspruch 1, wobei neben dem bioaktiven Mittel ein Hilfsstoff eingekapselt ist.

16. Mehrphasiges injizierbares Abgabesystem mit Langzeitfreisetzung nach Anspruch 15, wobei der Hilfsstoff Tyrosin, Polytyrosin, Dimethylglycin (DMG) oder Muramyldipeptid ist.

17. Mehrphasiges injizierbares Abgabesystem mit Langzeitfreisetzung nach Anspruch 1, wobei das eingekapselte bioaktive Mittel in wäßriger Form ist.

18. Mehrphasiges injizierbares Abgabesystem mit Langzeitfreisetzung nach Anspruch 1, wobei das eingekapselte bioaktive Mittel in lyophilisierter Form ist.

19. Mehrphasiges injizierbares Abgabesystem mit Langzeitfreisetzung nach Anspruch 1, wobei das makromolekulare bioaktiven Mittel ein nichtextrahiertes Allergenquellenmaterial ist.

20. Mehrphasiges injizierbares Abgabesystem mit Langzeitfreisetzung für längere, kontrollierte Abgabe eines mikroverkapselten Allergenextrakts, der einen Allergenextrakt umfaßt, eingekapselt in Mikrokapseln aus bioerodierbarem verkapselnden Polymer, das eine anhaltende, mehrphasige Freisetzung des Allergenextrakts gestattet, umfassend
(i) einen ersten Teil des Allergenextrakts, der nach Injektion aus den Mikrokapseln aus bioerodierbarem verkapselnden Polymer in einer Weise freisetzbar ist, daß nur eine relativ kleine Menge des Allergenextrakts im Laufe einer ersten Phase freigesetzt wird, wobei die einsetzende Allergenität aufgrund dessen minimiert wird, daß der erste Teil eine milde lokale Reaktion erzeugt, ähnlich der, die normalerweise bei niedrigen Dosen einer herkömmlichen Allergen-Verabreichung beobachtet wird; und
(ii) weitere Mengen Allergenextrakt, die eine wesentlich höhere Konzentration an Allergenextrakten in Dosen ergeben, die ohne die vorherige Freisetzung des ersten Teils eine ernstliche Reaktion am Patienten hervorrufen könnten.

21. Mehrphasiges injizierbares Abgabesystemmit Langzeitfreisetzung für längere, kontrollierte Abgabe eines mikroverkapselten Allergenextrakts nach Anspruch 20, wobei die Gesamtmenge an Allergenextrakt in den weiteren Mengen in höheren Konzentrationen sind als es eine toxische Konzentration für den Allergenextrakt ohne die vorherige Freisetzung des ersten Teils wäre.

22. Mehrphasiges injizierbares Abgabesystem mit Langzeitfreisetzung für längere, kontrollierte Abgabe eines mikroverkapselten Allergenextrakts nach Anspruch 20, wobei die wobei die schädlichen Wirkungen des freigesetzten Allergens kontrolliert werden durch die synchrone Freisetzung eines Mittels zur symptomatischen Behandlung einsetzender nachteiliger Reaktionen auf die anfängliche Dosis.

23. Mehrphasiges injizierbares Abgabesystem mit Langzeitfreisetzung nach Anspruch 22, wobei das Mittel ein entzündungshemmendes nichtsteroides Mittel, ein Antihistaminikum, ein Corticosteroid oder einen Bronchodilatator umfaßt.

24. Mehrphasiges injizierbares Abgabesystem mit Langzeitfreisetzung für längere, kontrollierte Abgabe eines mikroverkapselten Allergenextrakts nach Anspruch 20, wobei das bioerodierbare verkapselnde Polymer ein Vertreter ist, ausgewählt aus der Gruppe der Polylactide, Polyglycolide und deren Copolymeren und Derivaten; Nichtpeptid-Polyaminosäuren; Polyorthoester; nieder- und hochmolekularen Polyanhydride; Polyiminocarbonate; Poly-α-aminosäuren; Polyalkylcyanacrylate; Polyphosphazene; sowie Acyloxymethylpolyaspartate und Polyglutamat-Copolymere.

25. Mehrphasiges injizierbares Abgabesystem mit Langzeitfreisetzung für längere, kontrollierte Abgabe eines mikroverkapselten Allergenextrakts nach Anspruch 20, wobei der Allergenextrakt aus einer Allergenquelle stammt, ausgewählt aus der Gruppe bestehend aus Pollen, Schimmel, Lebensmittel, Tierschuppen, Tierausscheidungen, Schmutz, Insekten, Insektengifte und Insektenausscheidungen.

26. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung erreicht wird durch Bereitstellen einer heterogenen Mischung aus Mikrokugeln bioerodierbarer verkapselnder Polymere, wobei ein Teil der dem ersten Teil (i) entsprechenden Mikrokugeln eine Kombination aus einem oder mehreren der folgenden aufweist:
a. dünnere Allergen-imprägnierte Polymer-Schichten;
b. höhere Konzentrationen an Polymer;
c. niedrigere Konzentrationen an imprägniertem Allergen;
und ein Teil der den weiteren Mengen (ii) entsprechenden Mikrokugeln eine Kombination aus einem oder mehreren der folgenden aufweist:
a. dickere Allergen-imprägnierte Polymer-Schichten;
b. niedrigere Konzentrationen an Polymer;
c. unterschiedliche Verhältnisse an Copolymeren, die deren Erosionsgeschwindigkeit im Vergleich zum ersten Teil beeinflussen;
d. eine andere Polymerklasse mit einer ihr eigenen anderen Erosionsart oder -geschwindigkeit im Vergleich zum ersten Teil;
e. höhere Konzentrationen an imprägniertem Allergen.

27. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung homogen erreicht wird durch Verwendung nur einer der folgenden Art Mikrokugeln:
a. Mikrokugeln, die dickere Allergen-imprägnierte äußere Polymer-Schichten aufweisen und dünnere Allergenimprägnierte Polymer-Schichten im Kern; oder
b. Mikrokugeln, bei denen eine höhere Konzentration an Polymer in der äußeren Schichten der Mikrokugel als im Kern der Mikrokugel vorliegt; oder
c. Mikrokugeln, die unterschiedliche Verhältnisse an Copolymeren in den äußeren Schichten im Vergleich zum Kern aufweisen; oder
d. Mikrokugeln, bei denen die äußere Schicht aus einer Polymerklasse ist, und bei denen der Kern einer jeden Mikrokugel aus einer anderen Polymerklasse mit einer ihr eigenen anderen Erosionsart oder -geschwindigkeit ist; oder
e. Mikrokugeln, bei denen die äußere Schicht einer jeden Mikrokugel im Vergleich zum Kern einer jeden Mikrokugel eine niedrigere Konzentration an imprägniertem Allergen enthält, wodurch der Allergenextrakt des ersten Teils (i) in den äußeren Schichten enthalten ist, und der Allergenextrakt der weiteren Mengen (ii) im Kern enthalten ist.

28. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung erzielt wird durch Bereitstellen einer Mischung aus Mikrokugeln, wobei die dem ersten Teil (i) entsprechenden hydrolyseverzögernde Mittel in den Mikrokugel-Polymerschichten imprägniert umfassen, während die den weiteren Mengen (ii) entsprechenden Mikrokugeln hydrolysebeschleunigende Mittel oder gar keine beschleunigenden Mittel enthalten.

29. Mehrphasiges injizierbares Abgabesystem mit Langzeitfreisetzung nach Anspruch 28, wobei die hydrolyseverzögernden und -beschleunigenden Mittel nichttoxische organische Säuren oder Basen sind, oder ein saures, neutrales oder basisches anorganisches Salz oder eine Lösung derselben.

30. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung erzielt wird durch Bereitstellen einer Mischung aus Mikrokugeln, wobei die dem ersten Teil (i) entsprechenden keine hydrolyseverzögernden Mittel in den Mikrokugel-Polymerschichten imprägniert enthalten können, während die den weiteren Mengen (ii) entsprechenden Mikrokugeln hydrolysebeschleunigende Mittel enthalten.

31. Mehrphasiges injizierbares Abgabesystem mit Langzeitfreisetzung nach Anspruch 30, wobei die hydrolyseverzögernden und -beschleunigenden Mittel nichttoxische organische Säuren oder Basen sind, oder ein saures, neutrales oder basisches anorganisches Salz oder eine Lösung derselben.

32. Mehrphasiges injizierbares Abgabesystem mit Langzeitfreisetzung nach Anspruch 20, wobei neben dem Allergenextrakt ein Allergen-Stabilisierungsmittel eingekapselt ist.

33. Mehrphasiges injizierbares Abgabesystem mit Langzeitfreisetzung nach Anspruch 32, wobei das Allergen-Stabilisierungsmittel das Thermokonservierungsmittel Glycerin oder Mannit ist.

34. Mehrphasiges injizierbares Abgabesystem mit Langzeitfreisetzung nach Anspruch 20, wobei neben dem Allergenextrakt ein Hilfsstoff eingekapselt ist.

35. Mehrphasiges injizierbares Abgabesystem mit Langzeitfreisetzung nach Anspruch 34, wobei der Hilfsstoff Tyrosin, Polytyrosin, Dimethylglycin (DMG) oder Muramyldipeptid ist.

36. Mehrphasiges injizierbares Abgabesystem mit Langzeitfreisetzung nach Anspruch 20, wobei der eingekapselte Allergenextrakt in wäßriger Form ist.

37. Mehrphasiges injizierbares Abgabesystem mit Langzeitfreisetzung nach Anspruch 20, wobei das eingekapselte Allergen in lyophilisierter Form ist.

38. Mehrphasiges injizierbares Abgabesystemmit Langzeitfreisetzung nach Anspruch 20, wobei der eingekapselte Allergenextrakt physikalisch oder chemisch modifiziert ist.

39. Mehrphasiges injizierbares Abgabesystem mit Langzeitfreisetzung nach Anspruch 38, wobei niedermolekulare Allergen-Fraktionen von den physikalisch oder chemisch modifizierten Allergenen ausgeschlossen sind.

## Patentansprüche (Patentansprüche für folgende(n) Vertragsstaat(en): ES)

1. Mehrphasiges Abgabesystem mit Langzeitfreisetzung für längere, kontrollierte Abgabe eines mikroverkapselten makromolekularen bioaktiven Mittels, das ein makromolekulares bioaktives Mittel biologischen Ursprungs umfaßt, eingekapselt in Mikrokapseln aus bioerodierbarem verkapselnden Polymer, das eine anhaltende, mehrphasige Freisetzung des makromolekularen bioaktiven Mittels gestattet, umfassend
(i) einen ersten Teil des makromolekularen bioaktiven Mittels, der nach Injektion aus den Mikrokapseln aus bioerodierbarem verkapselnden Polymer in einer Weise freisetzbar ist, daß nur eine relativ kleine Menge des makromolekularen bioaktiven Mittels im Laufe einer ersten Phase freigesetzt wird, wobei die einsetzende ungünstige biologische Reaktion aufgrund dessen minimiert wird, daß der erste Teil eine milde Reaktion erzeugt, ähnlich der, die normalerweise bei niedrigen Dosen einer herkömmlichen Verabreichung beobachtet wird; und
(ii) weitere Mengen des makromolekularen bioaktiven Mittels, um eine wesentlich höhere Konzentration an makromolekularem bioaktiven Mittel in Dosen zu ergeben, die ohne die vorherige Freisetzung des ersten Teils eine ernstliche Reaktion am Patienten hervorrufen könnten.

2. Mehrphasiges Abgabesystem mit Langzeitfreisetzung nach Anspruch 1, wobei das makromolekulare bioaktive Mittel ausgewählt ist aus der Gruppe bestehend aus Allergenverwandten Substanzen, Cytokinen, Antihämophiliefaktoren und Rezeptor CD4.

3. Mehrphasiges Abgabesystem mit Langzeitfreisetzung nach Anspruch 1, wobei erster Teil und weitere Mengen des makromolekularen bioaktiven Mittels eine kontrollierte Freisetzung bereitstellen, die ein Dosierungsprofil mit beschleunigender oder zunehmender Freisetzung ergeben.

4. Mehrphasiges injizierbares Abgabesystem mit Langzeitfreisetzung für längere, kontrollierte Abgabe eines mikroverkapselten makromolekularen bioaktiven Mittels nach Anspruch 1, wobei die schädlichen Wirkungen des makromolekularen bioaktiven Mittels kontrolliert werden durch die synchrone Freisetzung eines Mittels zur symptomatischen Behandlung einsetzender nachteiliger Wirkungen der anfänglichen Dosis.

5. Mehrphasiges injizierbares Abgabesystem mit Langzeitfreisetzung nach Anspruch 4, wobei das Mittel zur symptomatischen Behandlung ein entzündungshemmendes nichtsteroides Mittel, ein Antihistaminikum, ein Corticosteroid oder einen Bronchodilatator umfaßt.

6. Mehrphasiges injizierbares Abgabesystem mit Langzeitfreisetzung für längere, kontrollierte Abgabe eines mikroverkapselten makromolekularen bioaktiven Mittels nach Anspruch 1, wobei das bioerodierbare verkapselnde Polymer ein Vertreter ist, ausgewählt aus der Gruppe der Polylactide, Polyglycolide und deren Copolymeren und Derivaten; Nichtpeptid-Polyaminosäuren; Polyorthoester; nieder- und hochmolekularen Polyanhydride; Polyiminocarbonate; Poly-α-aminosäuren; Polyalkylcyanacrylate; Polyphosphazene; sowie Acyloxymethylpolyaspartate und Polyglutamat-Copolymere.

7. Mehrphasiges injizierbares Abgabesystem mit Langzeitfreisetzung für längere, kontrollierte Abgabe eines mikroverkapselten bioaktiven Mittels nach Anspruch 1, wobei die Geschwindigkeit der mehrphasigen Freisetzung erreicht wird durch Bereitstellen einer heterogenen Mischung aus Mikrokugeln bioerodierbarer verkapselnder Polymere, wobei ein Teil der dem ersten Teil (i) entsprechenden Mikrokugeln eine Kombination aus einem oder mehreren der folgenden aufweist:
a. dünnere, mit bioaktivem Mittel imprägnierte Polymer-Schichten;
b. höhere Konzentrationen an Polymer;
c. niedrigere Konzentrationen an imprägniertem bioaktivem Mittel;
und ein Teil der den weiteren Mengen (ii) entsprechenden Mikrokugeln eine Kombination aus einem oder mehreren der folgenden aufweist:
a. dickere, mit bioaktivem Mittel imprägnierte Polymer-Schichten;
b. niedrigere Konzentrationen an Polymer;
c. unterschiedliche Verhältnisse an Copolymeren, die deren Erosionsgeschwindigkeit im Vergleich zum ersten Teil beeinflussen;
d. eine andere Polymerklasse mit einer ihr eigenen anderen Erosionsart oder -geschwindigkeit im Vergleich zum ersten Teil;
e. höhere Konzentrationen an imprägniertem bioaktiven Mittel.

8. Mehrphasiges injizierbares Abgabesystem mit Langzeitfreisetzung für längere, kontrollierte Abgabe eines mikroverkapselten Mittels nach Anspruch 1, wobei die Geschwindigkeit der mehrphasigen Freisetzung homogen erreicht wird durch Verwendung nur einer der folgenden Art Mikrokugeln:
a. Mikrokugeln, die dickere, mit bioaktivem Mittel imprägnierte äußere Polymer-Schichten aufweisen und dünnere, mit bioaktivem Mittel imprägnierte Polymer-Schichten im Kern; oder
b. Mikrokugeln, bei denen eine höhere Konzentration an Polymer in der äußeren Schichten der Mikrokugel als im Kern der Mikrokugel vorliegt; oder
c. Mikrokugeln, die unterschiedliche Verhältnisse an Copolymeren in den äußeren Schichten im Vergleich zum Kern aufweisen; oder
d. Mikrokugeln, bei denen die äußere Schicht aus einer Polymerklasse ist, und bei denen der Kern einer jeden Mikrokugel aus einer anderen Polymerklasse mit einer ihr eigenen anderen Erosionsart oder -geschwindigkeit ist; oder
e. Mikrokugeln, bei denen die äußere Schicht einer jeden Mikrokugel im Vergleich zum Kern einer jeden Mikrokugel eine niedrigere Konzentration an imprägniertem bioaktiven Mittel enthält, wodurch das bioaktive Mittel des ersten Teils (i) in den äußeren Schichten enthalten ist, und das bioaktive Mittel der weiteren Mengen (ii) im Kern enthalten ist.

9. Mehrphasiges injizierbares Abgabesystem mit Langzeitfreisetzung für längere, kontrollierte Abgabe eines mikroverkapselten bioaktiven Mittels nach Anspruch 1, wobei die Geschwindigkeit der mehrphasigen Freisetzung erzielt wird durch Bereitstellen einer Mischung aus Mikrokugeln, wobei die dem ersten Teil (i) entsprechenden hydrolyseverzögernde Mittel in den Mikrokugel-Polymerschichten imprägniert umfassen, während die den weiteren Mengen (ii) entsprechenden Mikrokugeln hydrolysebeschleunigende Mittel oder gar keine beschleunigenden Mittel enthalten.

10. Mehrphasiges injizierbares Abgabesystem mit Langzeitfreisetzung nach Anspruch 9, wobei die hydrolyseverzögernden und -beschleunigenden Mittel nichttoxische organische Säuren oder Basen sind, oder ein saures, neutrales oder basisches anorganisches Salz oder eine Lösung derselben.

11. Mehrphasiges injizierbares Abgabesystem mit Langzeitfreisetzung für längere, kontrollierte Abgabe eines mikroverkapselten bioaktiven Mittels nach Anspruch 1, wobei die Geschwindigkeit der mehrphasigen Freisetzung erzielt wird durch Bereitstellen einer Mischung aus Mikrokugeln, wobei die dem ersten Teil (i) entsprechenden keine hydrolyseverzögernden Mittel in den Mikrokugel-Polymerschichten imprägniert enthalten können, während die den weiteren Mengen (ii) entsprechenden Mikrokugeln hydrolysebeschleunigende Mittel enthalten.

12. Mehrphasiges injizierbares Abgabesystem mit Langzeitfreisetzung nach Anspruch 1, wobei die hydrolyseverzögernden und -beschleunigenden Mittel nichttoxische organische Säuren oder Basen sind, oder ein saures, neutrales oder basisches anorganisches Salz oder eine Lösung derselben.

13. Mehrphasiges injizierbares Abgabesystem mit Langzeitfreisetzung nach Anspruch 1, wobei neben dem bioaktiven Mittel ein Stabilisierungsmittel eingekapselt ist.

14. Mehrphasiges injizierbares Abgabesystem mit Langzeitfreisetzung nach Anspruch 13, wobei das Stabilisierungsmittel das Thermokonservierungsmittel Glycerin oder Mannit ist.

15. Mehrphasiges injizierbares Abgabesystem mit Langzeitfreisetzung nach Anspruch 1, wobei neben dem bioaktiven Mittel ein Hilfsstoff eingekapselt ist.

16. Mehrphasiges injizierbares Abgabesystem mit Langzeitfreisetzung nach Anspruch 15, wobei der Hilfsstoff Tyrosin, Polytyrosin, Dimethylglycin (DMG) oder Muramyldipeptid ist.

17. Mehrphasiges injizierbares Abgabesystem mit Langzeitfreisetzung nach Anspruch 1, wobei das eingekapselte bioaktive Mittel in wäßriger Form ist.

18. Mehrphasiges injizierbares Abgabesystem mit Langzeitfreisetzung nach Anspruch 1, wobei das eingekapselte bioaktive Mittel in lyophilisierter Form ist.

19. Mehrphasiges injizierbares Abgabesystem mit Langzeitfreisetzung nach Anspruch 1, wobei das makromolekulare bioaktiven Mittel ein nichtextrahiertes Allergenquellenmaterial ist.

20. Mehrphasiges injizierbares Abgabesystem mit Langzeitfreisetzung für längere, kontrollierte Abgabe eines mikroverkapselten Allergenextrakts, der einen Allergenextrakt umfaßt, eingekapselt in Mikrokapseln aus bioerodierbarem verkapselnden Polymer, das eine anhaltende, mehrphasige Freisetzung des Allergenextrakts gestattet, umfassend
(i) einen ersten Teil des Allergenextrakts, der nach Injektion aus den Mikrokapseln aus bioerodierbarem verkapselnden Polymer in einer Weise freisetzbar ist, daß nur eine relativ kleine Menge des Allergenextrakts im Laufe einer ersten Phase freigesetzt wird, wobei die einsetzende Allergenität aufgrund dessen minimiert wird, daß der erste Teil eine milde lokale Reaktion erzeugt, ähnlich der, die normalerweise bei niedrigen Dosen einer herkömmlichen Allergen-Verabreichung beobachtet wird; und
(ii) weitere Mengen Allergenextrakt, die eine wesentlich höhere Konzentration an Allergenextrakten in Dosen ergeben, die ohne die vorherige Freisetzung des ersten Teils eine ernstliche Reaktion am Patienten hervorrufen könnten.

21. Mehrphasiges injizierbares Abgabesystem mit Langzeitfreisetzung für längere, kontrollierte Abgabe eines mikroverkapselten Allergenextrakts nach Anspruch 20, wobei die Gesamtmenge an Allergenextrakt in den weiteren Mengen in höheren Konzentrationen sind als es eine toxische Konzentration für den Allergenextrakt ohne die vorherige Freisetzung des ersten Teils wäre.

22. Mehrphasiges injizierbares Abgabesystem mit Langzeitfreisetzung für längere, kontrollierte Abgabe eines mikroverkapselten Allergenextrakts nach Anspruch 20, wobei die wobei die schädlichen Wirkungen des freigesetzten Allergens kontrolliert werden durch die synchrone Freisetzung eines Mittels zur symptomatischen Behandlung einsetzender nachteiliger Reaktionen auf die anfängliche Dosis.

23. Mehrphasiges injizierbares Abgabesystem mit Langzeitfreisetzung nach Anspruch 22, wobei das Mittel ein entzündungshemmendes nichtsteroides Mittel, ein Antihistaminikum, ein Corticosteroid oder einen Bronchodilatator umfaßt.

24. Mehrphasiges injizierbares Abgabesystem mit Langzeitfreisetzung für längere, kontrollierte Abgabe eines mikroverkapselten Allergenextrakts nach Anspruch 20, wobei das bioerodierbare verkapselnde Polymer ein Vertreter ist, ausgewählt aus der Gruppe der Polylactide, Polyglycolide und deren Copolymeren und Derivaten; Nichtpeptid-Polyaminosäuren; Polyorthoester; nieder- und hochmolekularen Polyanhydride; Polyiminocarbonate; Poly-α-aminosäuren; Polyalkylcyanacrylate; Polyphosphazene; sowie Acyloxymethylpolyaspartate und Polyglutamat-Copolymere.

25. Mehrphasiges injizierbares Abgabesystem mit Langzeitfreisetzung für längere, kontrollierte Abgabe eines mikroverkapselten Allergenextrakts nach Anspruch 20, wobei der Allergenextrakt aus einer Allergenquelle stammt, ausgewählt aus der Gruppe bestehend aus Pollen, Schimmel, Lebensmittel, Tierschuppen, Tierausscheidungen, Schmutz, Insekten, Insektengifte und Insektenausscheidungen.

26. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung erreicht wird durch Bereitstellen einer heterogenen Mischung aus Mikrokugeln bioerodierbarer verkapselnder Polymere, wobei ein Teil der dem ersten Teil (i) entsprechenden Mikrokugeln eine Kombination aus einem oder mehreren der folgenden aufweist:
a. dünnere Allergen-imprägnierte Polymer-Schichten;
b. höhere Konzentrationen an Polymer;
c. niedrigere Konzentrationen an imprägniertem Allergen;
und ein Teil der den weiteren Mengen (ii) entsprechenden Mikrokugeln eine Kombination aus einem oder mehreren der folgenden aufweist:
a. dickere Allergen-imprägnierte Polymer-Schichten;
b. niedrigere Konzentrationen an Polymer;
c. unterschiedliche Verhältnisse an Copolymeren, die deren Erosionsgeschwindigkeit im Vergleich zum ersten Teil beeinflussen;
d. eine andere Polymerklasse mit einer ihr eigenen anderen Erosionsart oder -geschwindigkeit im Vergleich zum ersten Teil;
e. höhere Konzentrationen an imprägniertem Allergen.

27. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung homogen erreicht wird durch Verwendung nur einer der folgenden Art Mikrokugeln:
a. Mikrokugeln, die dickere Allergen-imprägnierte äußere Polymer-Schichten aufweisen und dünnere Allergen-imprägnierte Polymer-Schichten im Kern; oder
b. Mikrokugeln, bei denen eine höhere Konzentration an Polymer in der äußeren Schichten der Mikrokugel als im Kern der Mikrokugel vorliegt; oder
c. Mikrokugeln, die unterschiedliche Verhältnisse an Copolymeren in den äußeren Schichten im Vergleich zum Kern aufweisen; oder
d. Mikrokugeln, bei denen die äußere Schicht aus einer Polymerklasse ist, und bei denen der Kern einer jeden Mikrokugel aus einer anderen Polymerklasse mit einer ihr eigenen anderen Erosionsart oder -geschwindigkeit ist; oder
e. Mikrokugeln, bei denen die äußere Schicht einer jeden Mikrokugel im Vergleich zum Kern einer jeden Mikrokugel eine niedrigere Konzentration an imprägniertem Allergen enthält, wodurch der Allergenextrakt des ersten Teils (i) in den äußeren Schichten enthalten ist, und der Allergenextrakt der weiteren Mengen (ii) im Kern enthalten ist.

28. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung erzielt wird durch Bereitstellen einer Mischung aus Mikrokugeln, wobei die dem ersten Teil (i) entsprechenden hydrolyseverzögernde Mittel in den Mikrokugel-Polymerschichten imprägniert umfassen, während die den weiteren Mengen (ii) entsprechenden Mikrokugeln hydrolysebeschleunigende Mittel oder gar keine beschleunigenden Mittel enthalten.

29. Mehrphasiges injizierbares Abgabesystem mit Langzeitfreisetzung nach Anspruch 28, wobei die hydrolyseverzögernden und -beschleunigenden Mittel nichttoxische organische Säuren oder Basen sind, oder ein saures, neutrales oder basisches anorganisches Salz oder eine Lösung derselben.

30. Mehrphasiges injizierbares Abgabesystem mit Langzeitfreisetzung für längere, kontrollierte Abgabe eines mikroverkapselten Allergenextrakts nach Anspruch 20, wobei die Geschwindigkeit der mehrphasigen Freisetzung erzielt wird durch Bereitstellen einer Mischung aus Mikrokugeln, wobei die dem ersten Teil (i) entsprechenden keine hydrolyseverzögernden Mittel in den Mikrokugel-Polymerschichten imprägniert enthalten können, während die den weiteren Mengen (ii) entsprechenden Mikrokugeln hydrolysebeschleunigende Mittel enthalten.

31. Mehrphasiges injizierbares Abgabesystem mit Langzeitfreisetzung nach Anspruch 30, wobei die hydrolyseverzögernden und -beschleunigenden Mittel nichttoxische organische Säuren oder Basen sind, oder ein saures, neutrales oder basisches anorganisches Salz oder eine Lösung derselben.

32. Mehrphasiges injizierbares Abgabesystem mit Langzeitfreisetzung nach Anspruch 20, wobei neben dem Allergenextrakt ein Allergen-Stabilisierungsmittel eingekapselt ist.

33. Mehrphasiges injizierbares Abgabesystem mit Langzeitfreisetzung nach Anspruch 32, wobei das Allergen-Stabilisierungsmittel das Thermokonservierungsmittel Glycerin oder Mannit ist.

34. Mehrphasiges injizierbares Abgabesystem mit Langzeitfreisetzung nach Anspruch 20, wobei neben dem Allergenextrakt ein Hilfsstoff eingekapselt ist.

35. Mehrphasiges injizierbares Abgabesystem mit Langzeitfreisetzung nach Anspruch 34, wobei der Hilfsstoff Tyrosin, Polytyrosin, Dimethylglycin (DMG) oder Muramyldipeptid ist.

36. Mehrphasiges injizierbares Abgabesystem mit Langzeitfreisetzung nach Anspruch 20, wobei der eingekapselte Allergenextrakt in wäßriger Form ist.

37. Mehrphasiges injizierbares Abgabesystem mit Langzeitfreisetzung nach Anspruch 20, wobei das eingekapselte Allergen in lyophilisierter Form ist.

38. Mehrphasiges injizierbares Abgabesystem mit Langzeitfreisetzung nach Anspruch 20, wobei der eingekapselte Allergenextrakt physikalisch oder chemisch modifiziert ist.

39. Mehrphasiges injizierbares Abgabesystem mit Langzeitfreisetzung nach Anspruch 38, wobei niedermolekulare Allergen-Fraktionen von den physikalisch oder chemisch modifizierten Allergenen ausgeschlossen sind.

40. Verfahren zur Herstellung einer Langzeitfreisetzungs-Dosierungsform durch Einkapselung des bioaktiven Mittels der Ansprüche 1 bis 39 in Mikrokapseln aus bioerodierbarem verkapselnden Polymer.

## Revendications (Revendications pour l'(les) Etat(s) contractant(s) suivant(s): AT, BE, CH, LI, DE, DK, FR, GB, IT, LU, NL, SE)

1. Système d'administration à libération prolongée multiphasique pour une administration prolongée, contrôlée d'un agent bioactif macromoléculaire microencapsulé, comprenant un agent bioactif macromoléculaire d'une origine biologique, encapsulé dans des microcapsules en polymère encapsulant bioérodable, qui permet une libération prolongée, multiphasique, dudit agent bioactif macromoléculaire, incluant (i) une première portion dudit agent bioactif macromoléculaire qui est capable, après injection, de se libérer desdites microcapsules en polymère encapsulant bioérodable, de telle manière que seulement une quantité relativement petite dudit agent bioactif macromoléculaire est libérée durant une première phase, de sorte qu'une réaction biologique nuisible initiale est minimisée du fait que ladite première portion produit une réaction légère semblable à celle normalement observée avec de faibles doses d'une administration conventionnelle ; et (ii) des portions secondaires dudit agent bioactif macromoléculaire pour fournir un taux substantiellement plus élevé de l'agent bioactif macromoléculaire dans des doses qui pourraient, sans la libération antérieure de ladite première portion, provoquer une sérieuse réaction chez le patient.

2. Système d'administration à libération prolongée multiphasique selon la revendication 1, dans lequel l'agent bioactif macromoléculaire est choisi dans le groupe constitué des substances apparentées aux allergènes, des cytokines, des facteurs antihémophiliques et du récepteur CD4.

3. Système d'administration à libération prolongée multiphasique selon la revendication 1, dans lequel la première portion et les portions secondaires dudit agent bioactif macromoléculaire fournissent une libération prolongée fournissant un profil de dosage ayant une libération s'accélérant ou en crescendo.

4. Système d'administration injectable à libération prolongée multiphasique pour une administration prolongée, contrôlée d'un agent bioactif macromoléculaire microencapsulé selon la revendication 1, dans lequel les effets nocifs des agents bioactifs macromoléculaires libérés sont contrôlés par la libération synchrone d'un agent pour le traitement symptomatique de réactions initiales nuisibles, à la dose initiale.

5. Système d'administration injectable à libération prolongée multiphasique selon la revendication 4, dans lequel ledit agent pour le traitement symptomatique comprend un agent anti-inflammatoire non stéroïdien, un antihistaminique, un corticostéroïde, ou un bronchodilatateur.

6. Système d'administration injectable à libération prolongée multiphasique pour une administration prolongée, contrôlée d'un agent bioactif macromoléculaire microencapsulé selon la revendication 1, dans lequel ledit polymère encapsulant bioérodable est un élément choisi dans le groupe des poly(lactides), des poly(glycolides), et des copolymères et dérivés de ceux-ci ; des acides polyaminés non peptidiques ; des poly(ortho esters) ; des polyanhydrides de poids moléculaire bas et élevé ; des polyiminocarbonates ; des poly alpha-aminoacides ; du polycyano-acrylate d'alkyle ; des polyphosphazènes ; et du polyaspartate d'acyloxyméthyle et des copolymères de polyglutamate.

7. Système d'administration injectable à libération prolongée multiphasique pour une administration prolongée, contrôlée d'un agent bioactif microencapsulé selon la revendication 1, dans lequel la vitesse de libération multiphasique est obtenue en fournissant un mélange hétérogène de microsphères en polymères encapsulants bioérodables, une portion desdites microsphères correspondant à la première portion (i) ayant une combinaison d'une ou plusieurs des conditions suivantes :
a. des couches polymères, plus minces, imprégnées d'agent bioactif,
b. des concentrations plus élevées de polymère,
c. des concentrations plus basses d'agent bioactif imprégné,
et une portion desdites microsphères correspondant auxdites portions secondaires (ii) ayant une combinaison d'une ou plusieurs des conditions suivantes :
a. des couches polymères, plus épaisses, imprégnées d'agent bioactif,
b. des concentrations plus basses de polymère,
c. des rapports différents de copolymères, qui affectent leur vitesse d'érosion par rapport à la première portion,
d. une classe différente de polymère avec un moyen ou une vitesse de bioérosion intrinsèquement différents par rapport à la première portion,
e. des concentrations plus élevées d'agent bioactif imprégné.

8. Système d'administration injectable à libération prolongée multiphasique pour une administration prolongée, contrôlée d'un agent bioactif microencapsulé selon la revendication 1, dans lequel la vitesse de libération multiphasique est obtenue de façon homogène en utilisant seulement l'un des types suivants de microsphères :
a. des microsphères qui ont des couches polymères externes plus épaisses, imprégnées d'agent bioactif, et des couches polymères plus minces, imprégnées d'agent bioactif, dans le coeur, ou
b. des microsphères dans lesquelles il y a une concentration plus élevée de polymère dans les couches externes de la microsphère que dans le coeur de la microsphère, ou
c. des microsphères qui ont différents rapports de copolymères dans les couches externes par rapport au coeur, ou
d. des microsphères dans lesquelles la couche externe est réalisée en une classe de polymère, et dans lesquelles le coeur de chaque microsphère est réalisé en une classe différente de polymère, avec un moyen ou une vitesse de bioérosion intrinsèquement différents, ou
e. des microsphères dans lesquelles la couche externe de chaque microsphère contient une concentration plus faible d'agent bioactif imprégné par rapport au coeur de chaque microsphère, de sorte que l'agent bioactif de la première portion (i) soit contenu dans les couches externes et que l'agent bioactif des portions secondaires (ii) soit contenu dans le coeur.

9. Système d'administration injectable à libération prolongée multiphasique pour une administration prolongée, contrôlée d'un agent bioactif microencapsulé selon la revendication 1, dans lequel la vitesse de libération multiphasique est obtenue en fournissant un mélange de microsphères, celles correspondant à ladite première portion (i) incluant des agents retardant l'hydrolyse imprégnés dans les couches polymères des microsphères, tandis que les microsphères correspondant auxdites portions secondaires (ii) contiennent des agents accélérant l'hydrolyse, ou pas d'agent accélérant du tout.

10. Système d'administration injectable à libération prolongée multiphasique selon la revendication 9, dans lequel lesdits agents retardant et accélérant l'hydrolyse sont des acides ou des bases organiques non toxiques, ou un sel inorganique acide, neutre ou basique, ou une solution de ceux-ci.

11. Système d'administration injectable à libération prolongée multiphasique pour une administration prolongée, contrôlée d'un agent bioactif microencapsulé selon la revendication 1, dans lequel la vitesse de libération multiphasique est obtenue un fournissant un mélange de microsphères, celles correspondant à ladite première portion (i) peuvent ne pas inclure d'agents retardant l'hydrolyse imprégnés dans les couches polymères des microsphères, tandis que les microsphères correspondant auxdites portions secondaires (ii) contiennent des agents accélérant l'hydrolyse.

12. Système d'administration injectable à libération prolongée multiphasique selon la revendication 11, dans lequel lesdits agents retardant et accélérant l'hydrolyse sont des acides ou bases organiques non toxiques, ou un sel inorganique acide, neutre ou basique, ou une solution de ceux-ci.

13. Système d'administration injectable à libération prolongée multiphasique selon la revendication 1, dans lequel un agent stabilisant est encapsulé avec l'agent bioactif.

14. Système d'administration injectable à libération prolongée multiphasique selon la revendication 13, dans lequel ledit agent stabilisant est de la glycérine ou du mannitol thermoconservateur.

15. Système d'administration injectable à libération prolongée multiphasique selon la revendication 1, dans lequel un adjuvant est encapsulé avec l'agent bioactif.

16. Système d'administration injectable à libération prolongée multiphasique selon la revendication 15, dans lequel ledit adjuvant est la tyrosine, la polytyrosine, la diméthylglycine (DMG), ou le dipeptide muramyle.

17. Système d'administration injectable à libération prolongée multiphasique selon la revendication 1, dans lequel l'agent bioactif encapsulé est sous forme aqueuse.

18. Système d'administration injectable à libération prolongée multiphasique selon la revendication 1, dans lequel l'agent bioactif encapsulé est sous forme lyophilisée.

19. Système d'administration injectable à libération prolongée multiphasique selon la revendication 1, dans lequel l'agent bioactif macromoléculaire est un matériau source d'allergène non extrait.

20. Système d'administration injectable à libération prolongée multiphasique pour l'administration prolongée, contrôlée d'un extrait d'allergène microencapsulé comprenant un extrait d'allergène encapsulé dans des microcapsules en polymère encapsulant bioérodable, qui permet une libération prolongée, multiphasique, dudit extrait d'allergène, incluant (i) une première portion d'un extrait d'allergène qui, après injection, est capable de se libérer desdites microcapsules en polymère encapsulant bioérodable d'une manière telle que seulement une quantité relativement petite dudit extrait d'allergène est libérée durant une première phase, de sorte que l'allergénicité initiale est minimisée du fait que ladite première portion produit une légère réaction locale semblable à celle normalement observée avec de faibles doses d'une administration conventionnelle d'allergène ; et (ii) des portions secondaires d'extrait d'allergène qui fournissent un taux substantiellement plus élevé d'extraits d'allergène dans des doses qui pourraient, sans les libérations antérieures de ladite première portion, provoquer une sérieuse réaction chez le patient.

21. Système d'administration injectable à libération prolongée multiphasique pour l'administration prolongée, contrôlée d'un extrait d'allergène microencapsulé selon la revendication 20, dans lequel la quantité totale d'extrait d'allergène dans lesdites portions secondaires est à des taux plus élevés qu'un taux dudit extrait d'allergène qui serait toxique, en l'absence de la libération antérieure de ladite première portion.

22. Système d'administration injectable à libération prolongée multiphasique pour l'administration prolongée, contrôlée d'un extrait d'allergène microencapsulé selon la revendication 20, dans lequel les effets nocifs des allergènes libérés sont contrôlés par la libération synchrone d'un agent pour le traitement symptomatique de réactions initiales nuisibles, à la dose initiale.

23. Système d'administration injectable à libération prolongée multiphasique selon la revendication 22, dans lequel ledit agent comprend un agent anti-inflammatoire non stéroïdien, un antihistaminique, un corticostéroïde, ou un bronchodilatateur.

24. Système d'administration injectable à libération prolongée multiphasique pour l'administration prolongée, contrôlée d'un extrait d'allergène microencapsulé selon la revendication 20, dans lequel ledit polymère encapsulant bioérodable est un élément choisi dans le groupe des poly(lactides), des poly(glycolides), et des copolymères et dérivés de ceux-ce ; des acides polyaminés non peptidiques ; des poly(ortho esters) ; des polyanhydrides de poids moléculaire bas et élevé ; des polyiminocarbonates ; des poly alpha-aminoacides ; du polycyano-acrylate d'alkyle; des polyphosphazènes ; et du polyaspartate d'acyloxyméthyle et des copolymères de polyglutamate.

25. Système d'administration injectable à libération prolongée multiphasique pour l'administration prolongée, contrôlée d'un extrait d'allergène microencapsulé selon la revendication 20, dans lequel ledit extrait d'allergène est d'une source d'allergène choisie dans le groupe constitué du pollen, de la moisissure, des aliments, des phanères animaux, des excréments animaux, du fumeron, des insectes, du venin d'insecte et des excréments d'insectes.

26. Système d'administration injectable à libération prolongée multiphasique pour l'administration prolongée, contrôlée d'un extrait d'allergène microencapsulé selon la revendication 20, dans lequel la vitesse de libération multiphasique est obtenue en fournissant un mélange hétérogène de microsphères en polymères encapsulants bioérodables, une portion desdites microsphères correspondant à la première portion (i) ayant une combinaison d'une ou plusieurs des conditions suivantes :
a. des couches polymères, plus minces, imprégnées d'allergène,
b. des concentrations plus élevées de polymère,
c. des concentrations plus basses d'allergène imprégné,
et une portion desdites microsphères correspondant auxdites portions secondaires (ii) ayant une combinaison d'une ou plusieurs des conditions suivantes :
a. des couches polymères, plus épaisses, imprégnées d'allergène,
b. des concentrations plus basses de polymère,
c. des rapports différents de copolymères, qui affectent leur vitesse d'érosion par rapport à la première portion,
d. une classe différente de polymère avec un moyen ou une vitesse de bioérosion intrinsèquement différents par rapport à la première portion,
e. des concentrations plus élevées d'allergène imprégné.

27. Système d'administration injectable à libération prolongée multiphasique pour l'administration prolongée, contrôlée d'un extrait d'allergène microencapsulé selon la revendication 20, dans lequel la vitesse de libération multiphasique est obtenue de façon homogène en utilisant seulement l'un des types suivants de microsphères :
a. des microsphères qui ont des couches polymères externes plus épaisses, imprégnées d'allergène, et des couches polymères plus minces, imprégnées d'allergène, dans le coeur, ou
b. des microsphères dans lesquelles il y a une concentration plus élevée de polymère dans les couches externes de la microsphère que dans le coeur de la microsphère, ou
c. des microsphères qui ont différents rapports de copolymères dans les couches externes par rapport au coeur, ou
d. des microsphères dans lesquelles la couche externe est réalisée en une classe de polymère, et dans lesquelles le coeur de chaque microsphère est réalisé en une classe différente de polymère, avec un moyen ou une vitesse de bioérosion intrinsèquement différents, ou
e. des microsphères dans lesquelles la couche externe de chaque microsphère contient une concentration plus faible d'allergène imprégné par rapport au coeur de chaque microsphère, de sorte que l'extrait d'allergène de la première portion (i) soit contenu dans les couches externes et que l'extrait d'allergène des portions secondaires (ii) soit contenu dans le coeur.

28. Système d'administration injectable à libération prolongée multiphasique pour l'administration prolongée, contrôlée d'un extrait d'allergène microencapsulé selon la revendication 20, dans lequel la vitesse de libération multiphasique est obtenue en fournissant un mélange de microsphères, celles correspondant à ladite première portion (i) incluant des agents retardant l'hydrolyse imprégnés dans les couches polymères des microsphères, tandis que les microsphères correspondant auxdites portions secondaires (ii) contiennent des agents accélérant l'hydrolyse, ou pas d'agent accélérant du tout.

29. Système d'administration injectable à libération prolongée multiphasique selon la revendication 28, dans lequel lesdits agents retardant et accélérant l'hydrolyse sont des acides ou des bases organiques non toxiques, ou un sel inorganique acide, neutre ou basique, ou une solution de ceux-ci.

30. Système d'administration injectable à libération prolongée multiphasique pour l'administration prolongée, contrôlée d'un extrait d'allergène microencapsulé selon la revendication 20, dans lequel la vitesse de libération multiphasique est obtenue un fournissant un mélange de microsphères, celles correspondant à ladite première portion (i) peuvent ne pas inclure d'agents retardant l'hydrolyse imprégnés dans les couches polymères des microsphères, tandis que les microsphères correspondant auxdites portions secondaires (ii) contiennent des agents accélérant l'hydrolyse.

31. Système d'administration injectable à libération prolongée multiphasique selon la revendication 30, dans lequel lesdits agents retardant et accélérant l'hydrolyse sont des acides ou bases organiques non toxiques, ou un sel inorganique acide, neutre ou basique, ou une solution de ceux-ci.

32. Système d'administration injectable à libération prolongée multiphasique selon la revendication 20, dans lequel un agent stabilisant d'allergène est encapsulé avec l'extrait d'allergène.

33. Système d'administration injectable à libération prolongée multiphasique selon la revendication 32, dans lequel ledit agent stabilisant un allergène est de la glycérine ou du mannitol thermoconservateur.

34. Système d'administration injectable à libération prolongée multiphasique selon la revendication 20, dans lequel un adjuvant est encapsulé avec l'extrait d'allergène.

35. Système d'administration injectable à libération prolongée multiphasique selon la revendication 34, dans lequel ledit adjuvant est la tyrosine, la polytyrosine, la diméthylglycine (DMG), ou le dipeptide muramyle.

36. Système d'administration injectable à libération prolongée multiphasique selon la revendication 20, dans lequel l'extrait d'allergène encapsulé est sous forme aqueuse.

37. Système d'administration injectable à libération prolongée multiphasique selon la revendication 20, dans lequel l'allergène encapsulé est sous forme lyophilisée.

38. Système d'administration injectable à libération prolongée multiphasique selon la revendication 20, dans lequel l'extrait d'allergène encapsulé est physiquement ou chimiquement modifié.

39. Système d'administration injectable à libération prolongée multiphasique selon la revendication 38, dans lequel les fractions d'allergène de poids moléculaire bas sont exclues desdits allergènes physiquement ou chimiquement modifiés.

## Revendications (Revendications pour l'(les) Etat(s) contractant(s) suivant(s): ES)

1. Système d'administration à libération prolongée multiphasique pour une administration prolongée, contrôlée d'un agent bioactif macromoléculaire microencapsulé, comprenant un agent bioactif macromoléculaire d'une origine biologique, encapsulé dans des microcapsules en polymère encapsulant bioérodable, qui permet une libération prolongée, multiphasique, dudit agent bioactif macromoléculaire, incluant (i) une première portion dudit agent bioactif macromoléculaire qui est capable, après injection, de se libérer desdites microcapsules en polymère encapsulant bioérodable, de telle manière que seulement une quantité relativement petite dudit agent bioactif macromoléculaire est libérée durant une première phase, de sorte qu'une réaction biologique nuisible initiale est minimisée du fait que ladite première portion produit une réaction légère semblable à celle normalement observée avec de faibles doses d'une administration conventionnelle ; et (ii) des portions secondaires dudit agent bioactif macromoléculaire pour fournir un taux substantiellement plus élevé de l'agent bioactif macromoléculaire dans des doses qui pourraient, sans la libération antérieure de ladite première portion, provoquer une sérieuse réaction chez le patient.

2. Système d'administration à libération prolongée multiphasique selon la revendication 1, dans lequel l'agent bioactif macromoléculaire est choisi dans le groupe constitué des substances apparentées aux allergènes, des cytokines, des facteurs antihémophiliques et du récepteur CD4.

3. Système d'administration à libération prolongée multiphasique selon la revendication 1, dans lequel la première portion et les portions secondaires dudit agent bioactif macromoléculaire fournissent une libération prolongée fournissant un profil de dosage ayant une libération s'accélérant ou en crescendo.

4. Système d'administration injectable à libération prolongée multiphasique pour une administration prolongée, contrôlée d'un agent bioactif macromoléculaire microencapsulé selon la revendication 1, dans lequel les effets nocifs des agents bioactifs macromoléculaires libérés sont contrôlés par la libération synchrone d'un agent pour le traitement symptomatique de réactions initiales nuisibles, à la dose initiale.

5. Système d'administration injectable à libération prolongée multiphasique selon la revendication 4, dans lequel ledit agent pour le traitement symptomatique comprend un agent anti-inflammatoire non stéroïdien, un antihistaminique, un corticostéroïde, ou un bronchodilatateur.

6. Système d'administration injectable à libération prolongée multiphasique pour une administration prolongée, contrôlée d'un agent bioactif macromoléculaire microencapsulé selon la revendication 1, dans lequel ledit polymère encapsulant bioérodable est un élément choisi dans le groupe des poly(lactides), des poly(glycolides), et des copolymères et dérivés de ceux-ci ; des acides polyaminés non peptidiques ; des poly(ortho esters) ; des polyanhydrides de poids moléculaire bas et élevé ; des polyiminocarbonates ; des poly alpha-aminoacides ; du polycyano-acrylate d'alkyle ; des polyphosphazènes ; et du polyaspartate d'acyloxyméthyle et des copolymères de polyglutamate.

7. Système d'administration injectable à libération prolongée multiphasique pour une administration prolongée, contrôlée d'un agent bioactif microencapsulé selon la revendication 1, dans lequel la vitesse de libération multiphasique est obtenue en fournissant un mélange hétérogène de microsphères en polymères encapsulants bioérodables, une portion desdites microsphères correspondant à la première portion (i) ayant une combinaison d'une ou plusieurs des conditions suivantes :
a. des couches polymères, plus minces, imprégnées d'agent bioactif,
b. des concentrations plus élevées de polymère,
c. des concentrations plus basses d'agent bioactif imprégné,
et une portion desdites microsphères correspondant auxdites portions secondaires (ii) ayant une combinaison d'une ou plusieurs des conditions suivantes :
a. des couches polymères, plus épaisses, imprégnées d'agent bioactif,
b. des concentrations plus basses de polymère,
c. des rapports différents de copolymères, qui affectent leur vitesse d'érosion par rapport à la première portion,
d. une classe différente de polymère avec un moyen ou une vitesse de bioérosion intrinsèquement différents par rapport à la première portion,
e. des concentrations plus élevées d'agent bioactif imprégné.

8. Système d'administration injectable à libération prolongée multiphasique pour une administration prolongée, contrôlée d'un agent bioactif microencapsulé selon la revendication 1, dans lequel la vitesse de libération multiphasique est obtenue de façon homogène en utilisant seulement l'un des types suivants de microsphères :
a. des microsphères qui ont des couches polymères externes plus épaisses, imprégnées d'agent bioactif, et des couches polymères plus minces, imprégnées d'agent bioactif, dans le coeur, ou
b. des microsphères dans lesquelles il y a une concentration plus élevée de polymère dans les couches externes de la microsphère que dans le coeur de la microsphère, ou
c. des microsphères qui ont différents rapports de copolymères dans les couches externes par rapport au coeur, ou
d. des microsphères dans lesquelles la couche externe est réalisée en une classe de polymère, et dans lesquelles le coeur de chaque microsphère est réalisé en une classe différente de polymère, avec un moyen ou une vitesse de bioérosion intrinsèquement différents, ou
e. des microsphères dans lesquelles la couche externe de chaque microsphère contient une concentration plus faible d'agent bioactif imprégné par rapport au coeur de chaque microsphère, de sorte que l'agent bioactif de la première portion (i) soit contenu dans les couches externes et que l'agent bioactif des portions secondaires (ii) soit contenu dans le coeur.

9. Système d'administration injectable à libération prolongée multiphasique pour une administration prolongée, contrôlée d'un agent bioactif microencapsulé selon la revendication 1, dans lequel la vitesse de libération multiphasique est obtenue en fournissant un mélange de microsphères, celles correspondant à ladite première portion (i) incluant des agents retardant l'hydrolyse imprégnés dans les couches polymères des microsphères, tandis que les microsphères correspondant auxdites portions secondaires (ii) contiennent des agents accélérant l'hydrolyse, ou pas d'agent accélérant du tout.

10. Système d'administration injectable à libération prolongée multiphasique selon la revendication 9, dans lequel lesdits agents retardant et accélérant l'hydrolyse sont des acides ou des bases organiques non toxiques, ou un sel inorganique acide, neutre ou basique, ou une solution de ceux-ci.

11. Système d'administration injectable à libération prolongée multiphasique pour une administration prolongée, contrôlée d'un agent bioactif microencapsulé selon la revendication 1, dans lequel la vitesse de libération multiphasique est obtenue un fournissant un mélange de microsphères, celles correspondant à ladite première portion (i) peuvent ne pas inclure d'agents retardant l'hydrolyse imprégnés dans les couches polymères des microsphères, tandis que les microsphères correspondant auxdites portions secondaires (ii) contiennent des agents accélérant l'hydrolyse.

12. Système d'administration injectable à libération prolongée multiphasique selon la revendication 11, dans lequel lesdits agents retardant et accélérant l'hydrolyse sont des acides ou bases organiques non toxiques, ou un sel inorganique acide, neutre ou basique, ou une solution de ceux-ci.

13. Système d'administration injectable à libération prolongée multiphasique selon la revendication 1, dans lequel un agent stabilisant est encapsulé avec l'agent bioactif.

14. Système d'administration injectable à libération prolongée multiphasique selon la revendication 13, dans lequel ledit agent stabilisant est de la glycérine ou du mannitol thermoconservateur.

15. Système d'administration injectable à libération prolongée multiphasique selon la revendication 1, dans lequel un adjuvant est encapsulé avec l'agent bioactif.

16. Système d'administration injectable à libération prolongée multiphasique selon la revendication 15, dans lequel ledit adjuvant est la tyrosine, la polytyrosine, la diméthylglycine (DMG), ou le dipeptide muramyle.

17. Système d'administration injectable à libération prolongée multiphasique selon la revendication 1, dans lequel l'agent bioactif encapsulé est sous forme aqueuse.

18. Système d'administration injectable à libération prolongée multiphasique selon la revendication 1, dans lequel l'agent bioactif encapsulé est sous forme lyophilisée.

19. Système d'administration injectable à libération prolongée multiphasique selon la revendication 1, dans lequel l'agent bioactif macromoléculaire est un matériau source d'allergène non extrait.

20. Système d'administration injectable à libération prolongée multiphasique pour l'administration prolongée, contrôlée d'un extrait d'allergène microencapsulé comprenant un extrait d'allergène encapsulé dans des microcapsules en polymère encapsulant bioérodable, qui permet une libération prolongée, multiphasique, dudit extrait d'allergène, incluant (i) une première portion d'un extrait d'allergène qui, après injection, est capable de se libérer desdites microcapsules en polymère encapsulant bioérodable d'une manière telle que seulement une quantité relativement petite dudit extrait d'allergène est libérée durant une première phase, de sorte que l'allergénicité initiale est minimisée du fait que ladite première portion produit une légère réaction locale semblable à celle normalement observée avec de faibles doses d'une administration conventionnelle d'allergène ; et (ii) des portions secondaires d'extrait d'allergène qui fournissent un taux substantiellement plus élevé d'extraits d'allergène dans des doses qui pourraient, sans les libérations antérieures de ladite première portion, provoquer une sérieuse réaction chez le patient.

21. Système d'administration injectable à libération prolongée multiphasique pour l'administration prolongée, contrôlée d'un extrait d'allergène microencapsulé selon la revendication 20, dans lequel la quantité totale d'extrait d'allergène dans lesdites portions secondaires est à des taux plus élevés qu'un taux dudit extrait d'allergène qui serait toxique, en l'absence de la libération antérieure de ladite première portion.

22. Système d'administration injectable à libération prolongée multiphasique pour l'administration prolongée, contrôlée d'un extrait d'allergène microencapsulé selon la revendication 20, dans lequel les effets nocifs des allergènes libérés sont contrôlés par la libération synchrone d'un agent pour le traitement symptomatique de réactions initiales nuisibles, à la dose initiale.

23. Système d'administration injectable à libération prolongée multiphasique selon la revendication 22, dans lequel ledit agent comprend un agent anti-inflammatoire non stéroïdien, un antihistaminique, un corticostéroïde, ou un bronchodilatateur.

24. Système d'administration injectable à libération prolongée multiphasique pour l'administration prolongée, contrôlée d'un extrait d'allergène microencapsulé selon la revendication 20, dans lequel ledit polymère encapsulant bioérodable est un élément choisi dans le groupe des poly(lactides), des poly(glycolides), et des copolymères et dérivés de ceux-ce ; des acides polyaminés non peptidiques ; des poly(ortho esters) ; des polyanhydrides de poids moléculaire bas et élevé ; des polyiminocarbonates ; des poly alpha-aminoacides ; du polycyano-acrylate d'alkyle; des polyphosphazènes ; et du polyaspartate d'acyloxyméthyle et des copolymères de polyglutamate.

25. Système d'administration injectable à libération prolongée multiphasique pour l'administration prolongée, contrôlée d'un extrait d'allergène microencapsulé selon la revendication 20, dans lequel ledit extrait d'allergène est d'une source d'allergène choisie dans le groupe constitué du pollen, de la moisissure, des aliments, des phanères animaux, des excréments animaux, du fumeron, des insectes, du venin d'insecte et des excréments d'insectes.

26. Système d'administration injectable à libération prolongée multiphasique pour l'administration prolongée, contrôlée d'un extrait d'allergène microencapsulé selon la revendication 20, dans lequel la vitesse de libération multiphasique est obtenue en fournissant un mélange hétérogène de microsphères en polymères encapsulants bioérodables, une portion desdites microsphères correspondant à la première portion (i) ayant une combinaison d'une ou plusieurs des conditions suivantes :
a. des couches polymères, plus minces, imprégnées d'allergène,
b. des concentrations plus élevées de polymère,
c. des concentrations plus basses d'allergène imprégné,
et une portion desdites microsphères correspondant auxdites portions secondaires (ii) ayant une combinaison d'une ou plusieurs des conditions suivantes :
a. des couches polymères, plus épaisses, imprégnées d'allergène,
b. des concentrations plus basses de polymère,
c. des rapports différents de copolymères, qui affectent leur vitesse d'érosion par rapport à la première portion,
d. une classe différente de polymère avec un moyen ou une vitesse de bioérosion intrinsèquement différents par rapport à la première portion,
e. des concentrations plus élevées d'allergène imprégné.

27. Système d'administration injectable à libération prolongée multiphasique pour l'administration prolongée, contrôlée d'un extrait d'allergène microencapsulé selon la revendication 20, dans lequel la vitesse de libération multiphasique est obtenue de façon homogène en utilisant seulement l'un des types suivants de microsphères :
a. des microsphères qui ont des couches polymères externes plus épaisses, imprégnées d'allergène, et des couches polymères plus minces, imprégnées d'allergène, dans le coeur, ou
b. des microsphères dans lesquelles il y a une concentration plus élevée de polymère dans les couches externes de la microsphère que dans le coeur de la microsphère, ou
c. des microsphères qui ont différents rapports de copolymères dans les couches externes par rapport au coeur, ou
d. des microsphères dans lesquelles la couche externe est réalisée en une classe de polymère, et dans lesquelles le coeur de chaque microsphère est réalisé en une classe différente de polymère, avec un moyen ou une vitesse de bioérosion intrinsèquement différents, ou
e. des microsphères dans lesquelles la couche externe de chaque microsphère contient une concentration plus faible d'allergène imprégné par rapport au coeur de chaque microsphère, de sorte que l'extrait d'allergène de la première portion (i) soit contenu dans les couches externes et que l'extrait d'allergène des portions secondaires (ii) soit contenu dans le coeur.

28. Système d'administration injectable à libération prolongée multiphasique pour l'administration prolongée, contrôlée d'un extrait d'allergène microencapsulé selon la revendication 20, dans lequel la vitesse de libération multiphasique est obtenue en fournissant un mélange de microsphères, celles correspondant à ladite première portion (i) incluant des agents retardant l'hydrolyse imprégnés dans les couches polymères des microsphères, tandis que les microsphères correspondant auxdites portions secondaires (ii) contiennent des agents accélérant l'hydrolyse, ou pas d'agent accélérant du tout.

29. Système d'administration injectable à libération prolongée multiphasique selon la revendication 28, dans lequel lesdits agents retardant et accélérant l'hydrolyse sont des acides ou des bases organiques non toxiques, ou un sel inorganique acide, neutre ou basique, ou une solution de ceux-ci.

30. Système d'administration injectable à libération prolongée multiphasique pour l'administration prolongée, contrôlée d'un extrait d'allergène microencapsulé selon la revendication 20, dans lequel la vitesse de libération multiphasique est obtenue un fournissant un mélange de microsphères, celles correspondant à ladite première portion (i) peuvent ne pas inclure d'agents retardant l'hydrolyse imprégnés dans les couches polymères des microsphères, tandis que les microsphères correspondant auxdites portions secondaires (ii) contiennent des agents accélérant l'hydrolyse.

31. Système d'administration injectable à libération prolongée multiphasique selon la revendication 30, dans lequel lesdits agents retardant et accélérant l'hydrolyse sont des acides ou bases organiques non toxiques, ou un sel inorganique acide, neutre ou basique, ou une solution de ceux-ci.

32. Système d'administration injectable à libération prolongée multiphasique selon la revendication 20, dans lequel un agent stabilisant d'allergène est encapsulé avec l'extrait d'allergène.

33. Système d'administration injectable à libération prolongée multiphasique selon la revendication 32, dans lequel ledit agent stabilisant un allergène est de la glycérine ou du mannitol thermoconservateur.

34. Système d'administration injectable à libération prolongée multiphasique selon la revendication 20, dans lequel un adjuvant est encapsulé avec l'extrait d'allergène.

35. Système d'administration injectable à libération prolongée multiphasique selon la revendication 34, dans lequel ledit adjuvant est la tyrosine, la polytyrosine, la diméthylglycine (DMG), ou le dipeptide muramyle.

36. Système d'administration injectable à libération prolongée multiphasique selon la revendication 20, dans lequel l'extrait d'allergène encapsulé est sous forme aqueuse.

37. Système d'administration injectable à libération prolongée multiphasique selon la revendication 20, dans lequel l'allergène encapsulé est sous forme lyophilisée.

38. Système d'administration injectable à libération prolongée multiphasique selon la revendication 20, dans lequel l'extrait d'allergène encapsulé est physiquement ou chimiquement modifié.

39. Système d'administration injectable à libération prolongée multiphasique selon la revendication 38, dans lequel les fractions d'allergène de poids moléculaire bas sont exclues desdits allergènes physiquement ou chimiquement modifiés.

40. Procédé pour la préparation d'une forme de dosage à libération prolongée en encapsulant l'agent bioactif selon les revendications 1 à 39 dans des microcapsules en polymère encapsulant bioérodable.
